# EUROPÄISCHE PATENTSCHRIFT

(11) **EP 0 705 140 B1**
(45) Veröffentlichungstag und Bekanntmachung des Hinweises auf die Patenterteilung: **19.01.2000**
(21) Anmeldenummer: 93920820.3
(22) Anmeldetag: 24.09.1993
(51) Int. Cl.: B05B 11/00, B05B 7/04, B65D 83/16, A61M 15/00

(54) **AUSTRAGEINRICHTUNG FÜR FLIESSFÄHIGE MEDIEN**
DEVICE FOR DISCHARGING FREE FLOWING MEDIA
DISPOSITIF DE DECHARGE DE SUBSTANCES COULANTES

(43) Veröffentlichungstag der Anmeldung: 10.04.1996
(73) Patentinhaber: Ing. Erich Pfeiffer GmbH, 78315 Radolfzell (DE)
(72) Erfinder: ZUCKSCHWERDT, Friedrich, Wilhelm, D-78253 Eigeltingen (DE); JÄGER-WALDAU, Reinhold, D-78315 Radolfzell (DE)
(74) Vertreter: Patentanwälte Ruff, Beier, Schöndorf und Mütschele
(86) Internationale Anmeldenummer: EP9302602
(87) Internationale Veröffentlichungsnummer: WO9508399

(56) Entgegenhaltungen:
- WO-A-89/00085
- WO-A-89/00086
- DE-A- 4 011 537
- DE-A- 4 015 367
- DE-A- 4 102 632

## Beschreibung

Die Erfindung betrifft eine Austrageinrichtung nach dem Oberbegriff des Patentanspruchs 1. Sie ist insbesondere durch manuelles Umschließen frei und ggf. ohne von außen zugeführte Leitungen in einer Hand tragbar bzw. betätigbar. Die Austrageinrichtung soll zum Austrag eines einzigen oder mehrerer Medien geeignet sein, von denen das jeweilige flüssig, gasförmig, pulverförmig, pastös und/oder leichtflüchtig bzw. ähnlich sein kann.

Solche Austragvorrichtungen können z. B. gemäß der DE-OS-40 15 367 einen oder mehrere außen- bzw. innenliegende Austragköpfe aufweisen und dafür vorgesehen sein, mindestens ein Medium in möglichst feiner Verteilung auszubringen. Dies ist z. B. beim Ausbringen von Inhalations-Wirkstoffen erwünscht, um bei Asthma-Kranken eine möglichst schnelle Linderung bei geringster Dosierung zu erzielen. Feinste Verteilungen von nicht gasförmigen Wirkstoffen in einem Gas- bzw. Luft- oder Flüssigkeitsstrom als Trägerstrom sind aber auch auf vielen anderen Anwendungsgebieten erwünscht, z. B. dann, wenn zwei oder mehr gesonderte Wirkstoffe mit möglichst großer Homogenität vermischt und dabei bzw. dann ausgetragen werden sollen.

Verhältnismäßig leicht fließfähige Wirkstoffe können z. B. in ein oder mehreren Zerstäubungsstufen durch Zerstäuberdüsen, Wirbelkammern, Aufprallzerstäuber, Drallzerstäuber oder dgl. über einen Strömungsweg nach und nach feiner verteilt und stufenweise in feinere Partikelgrößen aufgeschlossen werden, jedoch werden hier meist Partikel- bzw. Tröpfchengrößen nicht unter 10µm erreicht.

Die DE-A-4 102 632 zeigt einen Düsenraum, in welchem das Medium feinst verteilt wird, allerdings ohne dabei verzögert bzw. in einen Stillstand überführt zu werden. Das Medium gelangt vielmehr in ununterbrochen kontinuierlicher Strömung in den verteilten Raum und von dort weiter zum Medienausgang.

Der Erfindung liegt des weiteren die Aufgabe zugrunde, eine Austrageinrichtung für Medien zu schaffen, mit welcher Nachteile bekannter Ausbildungen bzw. der beschriebenen Art vermieden werden können und die insbesondere eine besonders einfache Aufschließung mindestens eines nicht rein gasförmigen Mediums in feinste Verteilung und/oder kleinste Partikelgrößen gewährleistet.

Erfindungsgemäß sind die Merkmale nach Patentanspruch 1 vorgesehen. Im Falle einer nur aus einem einzigen oder mehreren Austragköpfen, z. B. Austragdüsen, bestehenden Austrageinrichtung, die zur Verbindung mit mindestens einem Medienspender vorgesehen ist, sind Mittel vorgesehen, durch welche wenigstens ein Teil mindestens einer für einen Austragvorgang vorgesehenen Austragcharge aus einem oder mehreren Medien in einer oder mehreren Medienvorlagen bereits in einer ausgebreiteten Verteilung nahezu in einem Ruhezustand bereitgestellt werden kann und durch welche dann das so bereitgestellte Medium einer weiteren, nochmals beginnenden Strömung ausgesetzt werden kann, durch welche das bereits fein verteilte Medium je nach den Erfordernissen in noch feinere Verteilung aufgeschlossen oder in geringere Verteilung überführt wird. Unter feiner Verteilung in der Medienvorlage ist hier insbesondere eine solche Verteilung zu verstehen, in welcher das Medium aufgrund seiner physikalischen Eigenschaften annähernd die dünnstmögliche, gerade noch zusammenhängende Schicht bildet, die im Falle einer Flüssigkeit einem Flüssigkeitsfilm entspricht und im Falle eines Pulvers bzw. Puders eine Dicke hat, die höchstens dem ein- bis zehn- bzw. höchstens zwanzigfachen der Partikelgröße entspricht. Unter Ruhezustand ist ein völliger Bewegungsstillstand in der Medienvorlage oder allenfalls eine Bewegung aufgrund einer Strömungsenergie zu verstehen, die kleiner ist als diejenige, welche erforderlich wäre, um das Medium aus der Austrageinrichtung ins Freie zu fördern.

Z. B. kann ein flüssiges Medium aufgrund seiner Kriechfähigkeit zu Beginn bzw. während der weiteren Strömung noch eine verhältnismäßig langsame Ausbreitungsbewegung ausführen, die erst zum Stillstand kommt, wenn sie ins Gleichgewicht mit der Haftspannung gelangt, mit welcher der Flüssigkeitsfilm an der entsprechenden Oberfläche der Medienvorlage haftet. Die Austrageinrichtung kann somit ähnlich einem Flächenvergaser arbeiten, wobei jedoch zweckmäßig nicht eine einzige, durchgehend glatte Fläche, sondern eine räumlich verteilte Flächenstruktur vorgesehen ist, deren einzelne Mikro-Flächenbereiche so verteilt sind und ineinander übergehen, daß sie von einem den Vorlagekörper durchströmenden Medium im wesentlichen allseits beaufschlagt werden. Dadurch läßt sich bei kleinstem Raumvolumen eine maximale Oberflächen-Ausdehnung als Haftgrundlage für das vorzulegende Medium erzielen.

Die Austragsverhältnisse können je nach Eigenschaften des Fluids, z. B. Viskosität, oder je nach gewünschten Austragcharakteristika in verhältnismäßig weitem Rahmen variiert werden, insbesondere hinsichtlich der sich ggf. während des Austrages ändernden Mengenverhältnisse zwischen vorzulegendem Medium und sekundärem Medium bzw. Gas. Die Mediumvorlage kann Z.B. nur etwa zu einem Drittel ihrer maximalen Aufnahmekapazität mit dem ersten Medium gefüllt werden. Es kann aber auch eine gegenüber dem ersten Medium wesentlich geringere Menge an sekundärem Medium in einem einzigen Austragvorgang gefördert werden, z. B. wenn an der Austrittsöffnung das Medium in Form eines geschlossenen Tropfens ins Freie austreten soll, anstatt in feinster Zerstäubung. Die Charakteristik der Strömung des Sekundärmediums kann dabei so gewählt werden, daß am Ende des Austrages des Primärmediums noch eine kleine Menge Sekundärmedium unter Druck so nachfließt, daß es eine eventuelle Verpfropfung der Austrittsöffnung durch das Primärmedium schlagartig völlig von der Austrittsdüse ablöst bzw. mit verhältnismäßig geringer Kraft ins Freie wegschleudert. Die Medienvorlage ist zweckmäßig gegen eines bis alle der Medien abweisend, z. B. im Falle eines wässrig gelösten Primärmediums wasserabweisend, so daß eine sehr energiearme Ablösung dieses Mediums von der Mediumvorlage gewährleistet ist. Z. B. kann die Mediumvorlage mit einer entsprechend abweisenden Beschichtung versehen sein, aus Polyurethanschaum bestehen oder ähnliches.

Bei entsprechend kleinen Durchlaßabständen zwischen benachbarten Oberflächen der jeweiligen Medien-Vorlage kann sich auch eine durchgehend vernetzte Kapillar-Struktur ergeben, durch die dafür gesorgt ist, daß ein wenigstens teilweise flüssiges Medium sich ohne von außen zugeführte Förderenergie nur aufgrund der Kapillarwirkung so fein wie möglich über einen entsprechend großen Bereich der Medienvorlage verteilt. Die Oberflächen der Medienvorlage können durch faserige, flächige, körnige, poröse und/oder ähnliche Strukturteile gebildet sein, die zweckmäßig eine Dicke von weit unter einem bzw. 1/10 oder 1/100 mm haben und jeweils in der Vernetzung an mindestens zwei, drei oder vier Seiten freiliegende Abschnitte bilden. Eine solche Medienvorlage kann z. B. durch einen porösen Hartkörper, einen Schaumstoff, ein Vlies, eine Packung aus Sieben und/oder ähnliches gebildet sein. Zweckmäßig bestehen die Benetzungsflächen der Medien-Vorlage teilweise oder insgesamt aus einem Werkstoff, der das Medium abstößt, so daß es dazu neigt, perlig zu haften.

Solche Körper sind z. B. auch zum Überführen schäumbarer Medien in Schaum geeignet, werden hier jedoch zumindest teilweise nicht zur Schaumerzeugung, sondern für solche Medien eingesetzt, die bei entsprechend hoher Strömungsgeschwindigkeit des Druckstoßes in voneinander gesonderten und daher fein verteilten Partikeln von den Oberflächen abgerissen und so weitergefördert werden.

Besonders vorteilhaft ist es, wenn Strukturteile der Medienvorlage unter den auftretenden Strömungskräften nicht lagestarr, sondern membran- bzw. lamellenartig rückfedernd beweglich sind, so daß sie Schwingungen ausführen können, die eine noch feinere Ablösung des vorgelegten Mediums, insbesondere von den Lamellenkanten, ermöglichen. Die minimalen Durchlaßquerschnitte der Mikrostruktur liegen dabei zweckmäßig weit oberhalb der größten, in dieser Verteilungsstufe zu erreichenden Partikelgröße, so daß die meisen Partikel nach dem Ablösen nicht wieder so aufeinandertreffen, daß sie sich zu größeren Partikeln verbinden können.

Die Beweglichkeit der Strukturteile ermöglicht auch das Entstehen von Schockwellen aufgrund des anfänglichen Druckstoßes mit kurzwellig zu- und abnehmender Strömungsgeschwindigkeit, die jedoch nach Strömungsbeginn im wesentlichen auch konstant bzw. sehr langsam stetig abnehmend sein kann. Der anfängliche Strömungsdruck kann dadurch über sehr lange Zeit im wesentlichen konstant gehalten werden, nämlich bis das Medium nahezu vollständig aus der Vorlage entfernt bzw. ausgetragen ist.

Der durch die Medienvorlage gebildeten Verteilungs- bzw. Zerstäubungsstufe kann eine oder mehrere solcher Stufen nachgeschaltet sein, von denen zweckmäßig die jeweilige als Beschleunigungsstufe, als Wirbelstufe, als Abreißstufe, als Aufprallstufe und/oder ähnliches für die Strömung ausgebildet ist. Die jeweilige Stufe kann teilweise oder ganz innerhalb der Medienvorlage oder außerhalb liegen, so daß z. B. zwei aufeinanderfolgende Stufen im Abstand voneinander liegen und in zeitlichem Abstand auf die Strömung wirken. Auch der Austritt der Strömung aus der Austrageinrichtung ins Freie am Medienausgang kann als Zerstäubungsstufe, nämlich als Zerstäubungsdüse ausgebildet sein, die im Verhältnis zur Strömungsgeschwindigkeit einen so kleinen Durchlaßquerschnitt bzw. so scharfe Begrenzungskanten aufweist, daß der Strömungsstrahl im wesentlichen über seinen gesamten Querschnitt verwirbelt und/oder als zerstäubter Kegelstrom freigegeben wird.

Die genannte Aufgabe läßt sich im wesentlichen unabhängig von der Ausbildung des Austragkopfes auch dadurch lösen, daß bei einer Austrageinrichtung, die zusätzlich zum jeweiligen Austragkopf einen Druckgasspeicher aufweist, ein eingeschlossenes Gasvolumen so auf einen erhöhten Druck vorgespannt werden kann, daß es nach Erreichen des Maximaldruckes im wesentlichen nur durch wegabhängige und nicht druckabhängige Freigabe als Trägerstrom schlagartig beginnend ausgetragen werden kann. Im Gegensatz zu einem reinen pneumatischen Energiespeicher oder einer druckabhängig öffnenden Luftpumpe wird hier also das Medium für einen Trägerstrom auf einen maximalen Druck vorgespannt und dann unter kontinuierlich schnellem bzw. möglichst geringen Druckabfall dem jeweiligen Medienausgang zugeführt, wobei es mindestens eine der genannten Verteilungs- bzw. Zerstäubungsstufen direkt oder indirekt beaufschlagen kann.

Bei einer bevorzugten Ausführungsform sind Mittel vorgesehen, um durch einen einzigen Betätigungsvorgang das Druckgasvolumen vorzuspannen und davor, währenddessen und/oder danach eine Austragcharge mindestens eines Wirkstoffes in wenigtens eine Medienvorlage zu überführen. Das vorgespannte Druckgasvolumen wird gegen Ausdehnung gesperrt und kann dann durch manuelles Auslösen der Sperre nur durch den jeweiligen Austragkopf hindurch entweichen, so daß es den dort bereits in feinster Verteilung vorgelegten Wirkstoff in der beschriebenen Weise ablöst, aufnimmt und zumindest am Düsenausgang in noch feinere Partikelgrößen aufschließt.

Die Austragvorrichtung kann langgestreckt mit einer größten Querschnittsweite von höchstens 100, 80 bzw. 50 mm und einer maximalen Länge von höchstens 250, 200 bzw. 150 mm sein, wobei im Falle eines Inhalationsgerätes das in den Mund einzuführende Austrag-Mundstück zweckmäßig so angeordnet und geformt ist, daß es eine zum Mund etwa parallele Lage der Austrageinrichtung bedingt. Das Mundstück selbst ist zweckmäßig nur ein in Längsansicht länglicher Hohlstutzen, der selbst nicht an der Strömungsführung teilnimmt, sondern durch den der Austragkopf vom hinteren Ende her im wesentlichen frei hindurchsprüht.

Diese und weitere Merkmale gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können. Ausführungsbeispiele der Erfindung sind in den Zeichnungen dargestellt und werden im folgenden näher erläutert. In den Zeichnungen zeigen:
- Fig. 1: eine Austrageinrichtung als Ausschnitt eines Austragkopfes,
- Fig. 2: einen Ausschnitt der Austrageinrichtung gem. Fig. 1 in Ansicht von rechts,
- Fig. 3: eine versorgungsautarke Gesamt-Austrageinrichtung mit einer Austragrichtung gem. den Figuren 1 und 2 in verkleinerter Ansicht,
- Fig. 4: die Austrageinrichtung gem. Fig. 3 in vergrößerter Darstellung und teilweise geschnitten,
- Fig. 5: einen Ausschnitt der Fig. 4 in vergrößerter Darstellung,
- Fig. 6: einen Querschnitt durch die Austragvorrichtung gem. Fig. 5,
- Fig. 7: einen Ausschnitt der Fig. 5 in nochmals vergrößerter Darstellung und in einer anderen Funktionsstellung,
- Fig. 8: einen Querschnitt durch die Austragvorrichtung gem. Fig. 5,
- Fig. 9: ein Detail der Fig. 4 in vergrößerter Darstellung und einer abgewandelten Ausbildung,
- Fig. 10: eine weitere Austrageinrichtung in einer Darstellung entsprechend Fig. 4,
- Fig. 11: einen Querschnitt durch die Austrageinrichtung gern. Fig. 10,
- Fig. 12: einen Querschnitt durch die Austrageinrichtung gem. Fig. 10,
- Fig. 13: ein Detail der Fig. 10 in einer abgewickelten
- Fig. 14: ein weiteres Detail der Fig. 10 in einer abgewickelten Darstellung,
- Fig. 15: die Austrageinrichtung gem. Fig. 10 in vergrößerter Darstellung und einer weiteren Funktionsstellung und
- Fig. 16: einen Ausschnitt in der Fig. 10 in weiter vergrösserter Darstellung.

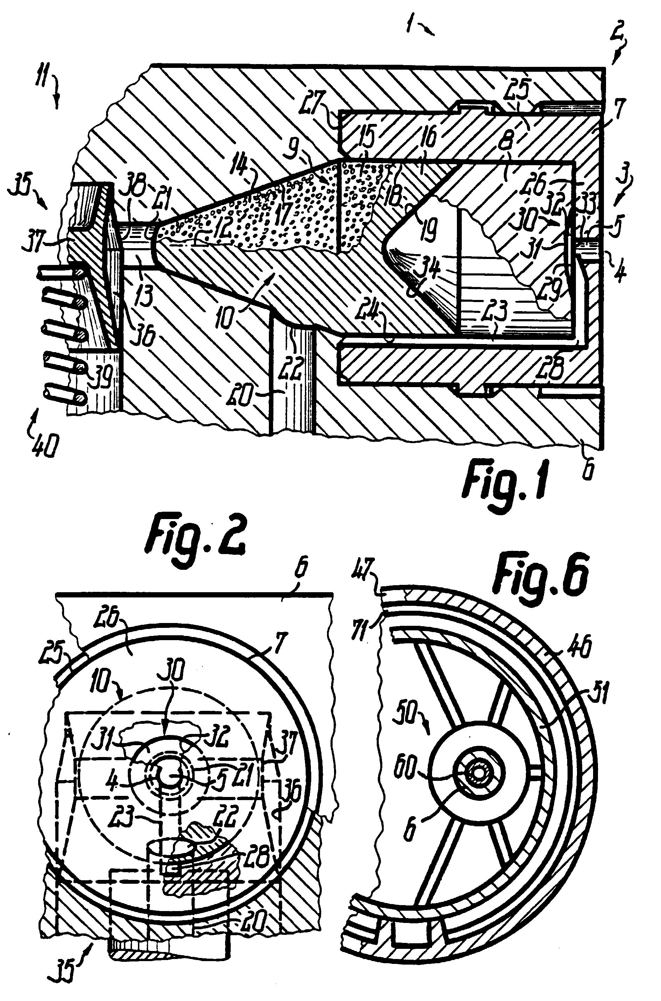

Die Austrageinrichtung nach den Figuren 1 und 2 ist im wesentlichen durch einen Austragkopf 2 bzw. eine Austragdüse 3 mit zugehörigen Leitungs- bzw. Kanalverbindungen gebildet und dafür vorgesehen, aus einem einzigen, annähernd geradlinig gerichteten Medienausgang 4 mindestens ein Medium so auszutragen, daß es an diesem Medienausgang 4 die Austrageinrichtung 1 verläßt. Der Medienausgang 4 ist durch einen kurzen Düsen- bzw. Endkanal 5 von einer Weite gebildet, die unterhalb einem bzw. ½ mm liegt. Zwischen seinen Enden bzw. bis zum Medienausgang 4 weist der glattwandige Endkanal 5 durchgehend annähernd konstante Durchlaßquerschnitte auf. Seine Länge liegt etwa in der Größenordnung des Ein- bis Dreifachen seiner Weite bzw. ist diese Länge kleiner als das Doppelte oder Dreifache dieser Länge, wobei beide Enden scharfkantig von Flanken begrenzt sind, die im Axialschnitt am inneren Ende annähernd rechtwinklig oder spitzwinklig zueinander liegen, falls der Kanal 5 nach außen erweitert ist. Zweckmäßig ist ein äußerster Längsabschnitt des Kanales 5, konisch unter etwa 90° bis 120° erweitert und somit gegenüber der engsten Weite des Kanales 5 kürzer; die Flanken am äußeren Ende liegen dann stumpfwinklig zueinander.

Die Austrageinrichtung 1 weist einen Grundkörper 6 mit einer Aufnahmeöffnung an der Medienaustrittseite auf, in die eine napfförmige Düsenkappe 7 so eingesetzt ist, daß sie mit dem Außenumfang ihres Mantels durch Kraftschluß und/oder formschlüssig durch eine Schnappverbindung feststehend lagegesichert ist und im wesentlichen vollständig versenkt innerhalb des Grundkörpers 6 liegt. Vollständig innerhalb der Düsenkappe 7 liegt ein Kernkörper 8, an dessen hinteres Ende ein aus der Düsenkappe 7 herausragender Füllkörper 9 anschließt.

Der Füllkörper 9 weist im wesentlichen über seine gesamte Erstreckung eine offenporig mikroporöse und in sich elastisch verschiebbare Kapillar- bzw. Netzstruktur aus durchgehend einteilig miteinander verbundenen Strukturteilen auf, die ähnlich einem Schaumstoffschwamm ein über alle Querschnitte und bis zu allen Peripherieflächen durchgehend zusammenhängendes, feinstes Kanal- und Kammersystem bilden. Durch Verformen bzw. Zusammendrücken oder innere Druckbeaufschlagung mit einem Fluid werden die Querschnitte, Volumina und/oder Formen dieser Mikrokanäle bzw. Mikrokammern verändert, nämlich vergrößert und/oder verkleinert sowie ggf. teilweise geschlossen. Gleichzeitig bildet diese Mikrostruktur an der gesamten Peripheriefläche der Medienvorlage 10 rasterartig feinst verteilte Mikroaustritte für das Medium, die jedoch über den weit überwiegenden Teil dieser Peripheriefläche durch Leit-Begrenzungsflächen bzw. Druckverdichtung des Strukturmateriales geschlossen sind.

Die Medienvorlage 10 bildet ein im Strömungsweg zwischen einer Fördereinrichtung 11 und dem Medienausgang 4 so im wesentlichen lagefest angeordnetes Kammersystem, daß alles von der Fördereinrichtung 11 geförderte Medium vor Erreichen des Medienausganges 4 die Medienvorlage 10 durchströmen muß. Anstatt seitlich versetzt oder im Winkel dazu liegt sie in einer Achse 12 der Austragdüse 3, in welcher auch der Medienausgang 4, die Düsenkappe 7 und der Kernkörper 8 etwa vorgesehen sind. Die Fördereinrichtung 11 ist über einen in dieser Achse 12 liegenden Druckstoß-Kanal 13 an die Medienvorlage 10 angeschlossen, dessen Durchlaßquerschnitt größer als der des Medienausganges 4 bzw. aller Kanalabschnitte ist, die zwischen der Medienvorlage 10 und dem Medienausgang 4 liegen.

Die Medienvorlage 10 schließt an den Kanal 13 und über dessen gesamte Weite mit einer Stirnfläche an, die etwa gleich groß wie der Querschnitt des Kanales 13 ist und von welcher aus die Medienvorlage 10 dadurch in ihren Querschnitten kontinuierlich zunimmt, daß sie hier einen spitzwinklig kegelstumpfförmigen Endabschnitt 14 bildet, dessen Länge mindestens so groß bzw. größer als seine mittlere Weite ist. An das weitere Ende des Abschnittes 14 schließt ein kurzer Abschnitt 15 konstanter Vollquerschnitte an, der am Außenumfang im wesentlichen zylindrisch begrenzt ist, gleiche Außenweite wie das weitere Ende des Abschnittes 14 hat und eine Länge aufweist, die zwischen etwa 1/3 und 1/4 seiner Außenweite liegt. An das vom Abschnitt 14 abgekehrte Ende dieses Abschnittes 15 schließt ein Abschnitt 16 an, der mit zunehmender Entfernung vom Abschnitt 15 abnehmende Ringquerschnitte dadurch aufweist, daß sein Innenumfang nach Art eines annähernd rechtwinkligen Spitzkegels mit kugelkalottenförmig abgerundeter Spitze zunimmt. Die Außenweite des Abschnittes 16 ist annähernd gleich derjenigen des Abschnittes 15, so daß diese beiden Abschnitte am Außenumfang kontinuierlich ineinander übergehen. Der Innenumfang des Abschnittes 16 geht kegelförmig in dessen Außenumfang über, so daß dessen Querschnitte auf Null abnehmen.

Jeder Abschnitt liegt über den größten Teil seines Umfanges und seiner Länge an hartwandigen Begrenzungen 17, 18, 19 des Grundkörpers 6, der Düsenkappe 7 bzw. des Kernkörpers 8 im wesentlichen vollflächig und unter Vorspannung an. Die am Außenumfang des Abschnittes 14 anliegende Begrenzung 17 des Grundkörpers 6 ist entsprechend kegelstumpfförmig und geht mit ihrem engeren Ende unmittelbar in das zugehörige Ende des Kanales 13 über. Das weitere Ende der Begrenzung 17 schließt annähernd an den Innenumfang der Düsenkappe 7 an, welcher die durchgehend im wesentlichen zylindrische Begrenzung 18 für den Außenumfang der beiden Abschnitte 15, 16 bildet. Auch an der vom Kanal 13 entfernten Seite liegt die Medienvorlage 10 mit ihrem stirnseitigen Innenumfang an einer Begrenzung 19 an, die durch das hintere, annähernd spitzkegelförmige Ende des Kernkörpers 8 gebildet ist. In entspanntem Zustand kann diese Stirnfläche der Medienvorlage 10 annähernd eben sein, so daß sie erst durch Eindrücken der Begrenzung 19 unter örtlicher Verdichtung der Mikrostruktur die beschriebene Form erhält. Entsprechend kann auch der Abschnitt 14 in entspanntem Zustand geringfügig größere Weiten oder einen größeren Kegelwinkel als die Begrenzung 17 haben, so daß sich zwischen der Spitze der Begrenzung 19 und dem Kanal 13 ein Kernbereich der Medienvorlage 10 ergibt, der gegenüber umgebenden Bereichen wenigstens geringfügig verdichtet ist.

Das den Ausgang des Kanales 13 überbrückende und gleiche Weite wie dieser Ausgang aufweisende engste Ende der Medienvorlage 10 bildet einen Druckstoß-Eingang 21. Da die Medienvorlage 10 hier nicht abgestützt ist, kann sie sich unter Entspannung in den Kanal 13 hineinwölben, so daß die am Eingang 21 liegenden Strukturöffnungen geringfügig aufgeweitet werden und sich ein entsprechend vergrößerter spezifischer Einlaßquerschnitt je Flächeneinheit ergibt. In den Außenumfang des Abschnittes 14 mündet quer zur Achse 12 ein wie der Kanal 13 im Grundkörper 6 vorgesehener Medien-Kanal 20, dessen durchgehend konstante Durchlaßquerschnitte etwa gleich denjenigen des Kanales 13 sein können und insofern in entsprechender Beziehung zu den übrigen Kanalabschnitten liegen. Der radiale Kanal 20 liegt näher beim Abschnitt 15 als beim Eingang 21 und durchsetzt die Begrenzung 17 so, daß er hier einen Medien-Eingang 22 bildet, welcher nach Art eines Verschlußes vom Außenumfang des Abschnittes 14 überbrückt ist. Der Durchlaßquerschnitt des Einganges 22, dessen Weite in der Größenordnung von einem Millimeter liegen kann, ist wesentlich kleiner als die größten, zur Durchströmung freien Durchlaßquerschnitte der Medienvorlage 10 und der Eingang 22 reicht nur über 1/5 bis 1/15, insbesondere 1/10 des Umfanges und nur etwa über 1/5 bis 1/3, insbesondere etwa 1/4 der Länge des Abschnittes 14. Auch hier entspannt sich die Medienvorlage 10 unter der beschriebenen Aufweitung der Mikrostruktur in den Kanal 20 hinein.

Die Außenumfänge der Abschnitte 15, 16 bilden annähernd über ihre gesamte gemeinsame Länge einen Vorlagen-Ausgang 24 für das Medium, der streifenförmig etwa parallel zur Achse 12 liegt und über den Außenumfang der Abschnitte 15, 16 nur über einen Teil reicht, der höchstens etwa so groß wie der des Einganges 22 bzw. demgegenüber um bis zu 1/3 kleiner ist. Der Ausgang 24 liegt etwa auf derselben Seite der Achse 12 bzw. der Medienvorlage 10 wie der Eingang 22 und zweckmäßig mit diesem bzw. dem Eingang 21 in einer gemeinsamen Axialebene.

Auch im Bereich des Ausganges 24 kann die Medienvorlage 10 entspannen und daher an der zugehörigen Oberfläche eine geringfügig aufgeweitete Mikrostruktur bilden. Der Ausgang 24 erstreckt sich annähernd vom weiteren Ende des Abschnittes 14 bis zum weiteren Ende der Begrenzung 19, wobei auch zwei oder mehr annähernd gleichmäßig über den Umfang verteilte Ausgänge 24 vorgesehen sein können.

Der Ausgang 24 ist durch ein Ende eines Düsenkanales 23 gebildet, der seinerseits durch eine Längsnut am Innenumfang des Mantels 25 der Düsenkappe 7 gebildet ist und von der Innenseite von deren Stirnwand 26 bis zum hinteren Ende 27 des Mantels 25 durchgeht. An der offenen Nutlängsseite ist die Längsnut im Bereich des Ausganges 24 von der Medienvorlage 10 und unmittelbar anschließend vom Außenumfang des Kernkörpers 8 verschlossen, so daß der Düsenkanal 23 über seine gesamte Länge einen über den Umfang geschlossenen Kanal bildet. Dessen durchgehend im wesentlichen konstante Durchlaßquerschnitte sind wesentlich kleiner als die des Einganges 21 sowie als die demgegenüber zunehmenden und größeren Durchlaßquerschnitte der Abschnitte 14, 15 und eines anschließenden Teiles des Abschnittes 16. Dadurch ergibt sich aus der Medienvorlage 10 heraus in den Medienausgang 24 und in den Düsenkanal 23 eine wesentliche Strömungsbeschleunigung und eine starke Strömungsverwirbelung.

Mit seiner von der Medienvorlage 10 abgekehrten, anschließend an den Außenumfang ringscheibenförmig annähernd ebenen Stirnfläche liegt der Kernkörper 8 annähernd vollflächig an der Innenseite der Stirnwand 26 permanent lagefest an. Diese Stirnfläche ist durch einen Endabschnitt von annähernd konstanter Außenweite des Kernkörpers 8 gebildet und an das hintere Ende dieses Abschnittes schließt der die Begrenzung 19 bildende, kürzere Abschnitt an, wobei die schräge Begrenzung 19 bis zum Außenumfang des vorderen Endabschnittes reicht. Der Längsabschnitt des Düsenkanales 23 geht am vorderen Ende über eine annähernd rechtwinklige Umlenkung 28 in einen etwa radial zur Achse 12 liegenden kürzeren Querabschnitt über, der durch eine entsprechende Nut an der Innenseite der Stirnwand 26 gebildet und an der offenen Nutlängsseite durch die Stirnfläche des Kernkörpers 8 verschlossen ist. Die Breite dieses Querabschnittes kann etwa gleich derjenigen des Längsabschnittes und die Tiefe etwas größer sein, so daß sich hier je nach den Erfordernissen geringfügig vergrößerte oder verringerte Durchlaßquerschnitte bzw. entsprechend eine geringfügige Reduzierung oder Erhöung der Strömungsgeschwindigkeit ergeben. Das vom Längsabschnitt entfernte Ende des Querabschnittes bildet eine gegen die vordere Stirnfläche des Kernkörpers 8 gerichtete Richtdüse 29, aus welcher der Medienstrom wenigstens teilweise entgegen der Hauptströmungs-bzw. Austragrichtung der Austragdüse 3 und/oder wenigstens teilweise quer bzw. schräg zu dieser Richtung austritt. Der Auslaßquerschnitt dieser Richtdüse 29 kann etwa in der Grössenordnung der Durchlaßquerschnitte des Endkanales 5 liegen.

Im Bereich des Austrittes der Richtdüse 29 bzw. des inneren Endes des Endkanales 5 ist ein Prallzerstäuber 30 vorgesehen, gegen dessen napfförmige vertiefte Prallfläche 31 die Richtdüse 29 gerichtet ist. Die Prallfläche 31 ist als zentrale bzw. etwa in der Achse 12 liegende Vertiefung in der vorderen Stirnseite des Kernkörpers 8 vorgesehen und schließt mit dem Außenumfang ihres stumpfwinklig kegelstumpfförmigen Mantels an den Innenumfang der zugehörigen ringförmigen Stirnfläche an. Die Bodenfläche der Prallfläche 31 ist annähernd eben, liegt etwa rechtwinklig zur Achse 12 und reicht in Ansicht parallel zur Achse 12 über den Innenumfang des inneren Endes des Endkanales 5 hinaus. Durch die Prallfläche 31 und die diese deckelartig verschließende Innenseite der Stirnwand 26 ist eine spaltartig dünne Scheiben- bzw. Wirbelkammer 32 gebildet, deren größte Dicke in der Größenordnung von nur einem oder wenigen zehntel oder darunter bei etwa einem halben zehntel Millimeter liegt, wobei diese Dicke zum Außenumfang stetig bis auf Null abnimmt. Diese Wirbelkammer 32 ist nur an einem einzigen, etwa in ihrer Mittelachse liegenden Ausgang 33 offen, der durch das innere Ende des Endkanales 5 gebildet sowie gegenüber der Weite der Wirbelkammer 32 weniger als 1/2 bzw. 1/3 so weit ist. Unmittelbar an den Außenumfang dieses Ausganges 33 grenzt auch die Richtdüse 29 an, die diesen Außenumfang entweder nicht oder nur auf der zugehörigen Seite der Achse 12 geringfügig durchsetzen kann, so daß ein Teil des aus der Richtdüse 29 austretenden Medienstromes quer über den Ausgang 33 und entlang der Innenseite der Stirnwand 26 gerichtet ist. Im Querschnitt kann die Begrenzung der Richtdüse 29 mit derjenigen des Ausganges 33 eine scharfe bzw. spitzwinklige Spitze bilden, die etwa in der Ebene des übrigen Ausganges 33 liegt. Dadurch können hier Verwirbelungen noch verstärkt werden. Die Wirbelkammer 32 ist von Innenkörpern frei, liegt hinter dem Endkanal 5 bzw. der Stirnwand 26 und hat nach Art eines Parabolspiegels eine die Strömung auf den Ausgang 33 fokusierende bzw. reflektierende Wirkung, wobei der in den Ausgang 33 abprallende Strahl den aus der Richtdüse 29 eintretenden Strahl durchbricht.

Auch die Begrenzungen 17, 18, 19 bilden Leitflächen für das Medium, wobei insbesondere die Begrenzung 19 eine Prall- bzw. Leitfläche 34 bilden kann, durch welche das Medium zum Ausgang 24 hin geleitet wird. Durch die Querschnittsform des Abschnittes 16 bildet die Medienvorlage 10 sich trichterartig zum Ausgang 24 verjüngende Querschnitte, wobei ihre spezifischen Struktur-Durchlaßquerschnitte zum Ausgang 24 weiter werden und am Medienausgang 24 eine rasterartige Verteilung von Mikroöffnungen gebildet ist, die im wesentlichen von freiliegenden Strukturteilen begrenzt bzw. membran- oder faserartig voneinander getrennt sind. Dadurch ergeben sich mit zunehmender Entfernung von der Leitfläche 24 bzw. den Begrenzungen 17, 18 geringere Strömungswiderstände, jedoch zum Ausgang 24 zunehmende Strömungsgeschwindigkeiten innerhalb der Medienvorlage 10.

Je nach Viskosität und anderen physikalischen Eigenschaften des auszubringenden Mediums könnte die Vorlage auch nur durch Begrenzungen eines Hohlraumes, beispielsweise entsprechend den Begrenzungen 17, 18, 19 gebildet und daher im wesentlichen flächig sein. Wegen weiterer Merkmale und Wirkungen einer solchen Austrageinrichtung wird auf die DE 41 02 632 A Bezug genommen.

Zur Beeinflussung bzw. Steuerung des bevorzugt gesondert vom Kanal 20 der Medienvorlage 10 nur durch den Kanal 13 zugeführten Druckstoßes ist ein Ventil 35 vorgesehen, dessen Hauptachse zweckmäßig quer bis rechtwinklig zur Achse 12 und/oder mit dieser in einer gemeinsamen Axialebene liegt. Das Ventil 35 weist in einer an eine Druckquelle angeschlossenen Ventilkammer 36 einen axial verschiebbaren Ventilkörper 37 auf, der mit zwei voneinander abgekehrten, kegelstumpfförmigen Dichtlippen abgedichtet am Innenumfang der Ventilkammer 36 läuft. Dieser Innenumfang ist von einem Kammerausgang 38 durchsetzt, welcher durch das hintere Ende des Kanales 13 gebildet ist und einen Abstand von dem Eingang 21 hat, der höchstens das drei- bis fünffache der größten Weite des Kanales 13 beträgt bzw. etwa in der Größenordnung dieser Weite liegt oder kleiner als diese Weite ist. Der Kammerausgang 38 ist in Ausgangsstellung des Ventiles 35 durch den Ventilkörper 37 geschlossen, wobei er zwischen dessen Ventillippen liegt und der Ventilkörper 37 zur anschlagbegrenzten Ausgangsstellung mit einer Ventilfeder 39 belastet ist. Wegen weiterer Merkmale und Wirkungen des Ventiles 35 sowie von dessen Anordnung wird auf die DE 40 11 537 A Bezug genommen. Das Ventil 35 ist Bestandteil von Steuermitteln 40, durch welche die bestimmungsgemäße Funktionsfolge der Austrageinrichtung 1 festgelegt sind.

Nach dieser Funktionsfolge wird zuerst über den Kanal 20 und den Eingang 22 der Medienvorlage 10 ein Tropfen flüssigen Wirkstoffes zugeführt, der sich aufgrund der Kapillarwirkung vom Eingang 22 in das Innere der Medienvorlage 10 ausbreitet, wobei die Ausbreitung auch in Richtung zum Eingang 21 und zum Ausgang 24 erfolgt. Das dosierte Volumen an Wirkstoff kann unterhalb der Sättigungsgrenze der Medienvorlage 10 liegen bzw. so gering sein, daß die Kapillarwirkung beendet ist, bevor sich der Wirkstoff über das gesamte Volumen der Medienvorlage 10 bzw. bis zu den dem Eingang 22 gegenüberliegenden Bereichen der Begrenzung 17, 18 ausgebreitet hat. Zu Beginn, während und/oder nach Abschluß dieser Ausbreitung wird das Ventil 35 dadurch geöffnet, daß die eine Dichtlippe des Ventilkörpers 37 über den Kammerausgang 38 hinaus verschoben wird. Dadurch steht der Kammerausgang 38 in unmittelbarer Leitungsverbindung mit demjenigen Teil der Ventilkammer 36, der permanent mit einer Druckkammer leitungsverbunden ist, die in dieser Funktionsphase unter maximalem Druck steht und deren maximales Aufnahmevolumen mindestens 100- bis 200-fach so groß wie das der Medienvorlage 10 oder der Ventilkammer 36 ist.

Es ist zwar denkbar, den Druckstoß wenigstens teilweise mit einem flüssigen bzw. leicht flüchtigen oder verdampfenden Medium, ggf. durch den Kanal 22, durchzuführen, jedoch erfolgt er bevorzugt durch ein kompressibles Medium, wie Luft oder ein anderes Gas, und ausschließlich über den Eingang 21, während der Eingang 22 in wesentlichen verschlossen ist, z. B. durch Ventilverschluß des Kanales 20 oder dadurch, daß seine Enge in Bezug auf die Strömungsquerschnitte in der Medienvorlage 10 ähnlich einem Verschluß wirkt; auch eine Flüssigkeitssäule im Kanal 20 kann als Verschluß vorgesehen sein. Durch teilweises oder vollständiges bzw. allmähliches oder ruckartig schnelles Öffnen des Kammerausganges 38 unabhängig von irgendeinem gleichzeitig erfolgenden Druckanstieg in der Druckkammer der Fördereinrichtung 11 strömt das Druckstoß-Medium mehr oder weniger schlagartig und mit sehr hoher Strömungsgeschwindigkeit gegen den Eingang 21. Dieses Medium kann in der Druckkammer z. B. auf mindenstens 1/5 oder 1/10 seines entspannten Volumens komprimiert sein. Das Medium durchströmt dann die wenigstens teilweise nur oberflächig benetzte und/oder wenigstens teilweise gefüllte Kanalstruktur der Medienvorlage 10 mit einer Strömungsgeschwindigkeit, die je nach Sättigungsgrad der Medienvorlage 10 etwa gleich groß, größer oder abnehmend kleiner als die Strömungsgeschwindigkeit am Eingang 21 sein kann.

Da das Druckstoß-Medium prallartig auf den Eingang 21 und im Inneren der Mediumvorlage 10 auf die Strukturteile und den vorgelegten Wirkstoff auftrifft, entstehen in der Medienvorlage 10 Schwingungen dieser Strukturteile, welche zusätzlich zur Abreißen durch die Strömung zum Ablösen des Wirkstoffes in sehr feinen Tröpfchen führen. Diese werden vom Trägerstrom in der Medienvorlage 10 auf zahlreichen, im wesentlichen zick-zack-förmigen Wegen mitgenommen, die sich aus der Art der Mikrostruktur ergeben und deren begradigte Hauptrichtung zum Ausgang 24 führt. Durch die Strukturverdichtungen im Bereich der Begrenzungen 17, 18, 19 neigt die Strömung nicht dazu, diese Bereiche zu erreichen, sondern sie verbleibt im wesentlichen mit Abstand innerhalb der Peripherie-Flächen der Medienvorlage 10. Im Bereich des Abschnittes 16 kann die Strömung in eine kreis- bzw. ringförmige Rotationsströmung um die Achse 12 versetzt und dadurch dem Ausgang 24 zugeführt werden.

Eventuell bis zur Leitfläche 34 vordringende Teile der Strömung werden entlang dieser Leitfläche unmittelbar zum vorderen Ende des Ausganges 24 in den Düsenkanal 23 geleitet. Im Bereich dieses vorderen Endes kann die Austritts-Strömungsgeschwindigkeit dadurch am größten sein und nach hinten abfallen. Das hintere Ende der Begrenzung 19 liegt im Abstand vor dem hinteren Ende des Ausganges 24 bzw. des Abschnittes 15 etwa in der Mitte zwischen dem Abschnitt 14 und dem vorderen Ende des Ausganges 24. Der Übergang zwischen den Abschnitten 14, 15 kann etwa in der Ebene des hinteren Endes 27 liegen und die Länge des Ausganges 24 kann mindestens drei- bis achtfach, insbesondere etwa sechs- bis siebenfach länger als die größte zugehörige Weite des Düsenkanales 23 bzw. von dessen Längsabschnitt sein, welcher länger als der Ausgang 24, z. B. etwa doppelt so lang ist. Der Querabschnitt des Düsenkaneles 23 ist demgegenüber bzw. gegenüber dem Ausgang 24 kürzer.

Der Wirkstoff verläßt den Ausgang 24 in Form feinstverteilter Tröpfchen innerhalb des Trägerstromes, der am Übertritt in den Düsenkanal 23 gegenüber der Strömungsgeschwindigkeit am Eingang hoch beschleunigt wird. Der Strom fließt dabei entlang der von stumpfwinkligen Flanken begrenzten Umfangskante zwischen der Begrenzung 19 und dem anschließenden Abschnitt des Kernkörpers 8, in deren Bereich eine weitere Verwirbelung erfolgt, aufgrund deren und der hohen Strömungsgeschwindigkeit die Tröpfchengröße hier bzw. im Düsenkanal 23 und an der Umlenkung 28 noch weiter verkleinert werden kann. Die Fluidströmung prallt dann aus der Richtdüse 29 mit hoher Geschwindigkeit unter unterschiedlich spitzen und stumpfen Winkeln auf die Prallfläche 31, von der sie teilweise direkt in den Ausgang 33 und teilweise gegen die diese umgebende Stirn- bzw. Ringfläche der Wirbelkammer 32 reflektiert wird. Durch den Stau am Ausgang 33 entsteht in der Wirbelkammer 32 ein verhältnismäßig hoher Überdruck, unter welchem der Fluidstrom aus der Wirbelkammer 32 durch den Ausgang 33 und entlang von dessen scharfer Kante ausströmt. Dadurch wird nicht nur durch den ein- oder mehrfach hin- und hergehenden Aufprall in der Wirbelkammer 32, sondern auch durch Strömungsabscherung an der Kante des Ausganges 33 die Tröpfchengröße noch weiter verringert. Vom höchsten Druck in der Wirbelkammer 32 fällt der Druck vom Ausgang 33 zum Medienausgang 4 ab. Dies stellt im wesentlichen den größten Druckunterschied in der gesamten Medienführung dar. Im Abstand danach erfolgt eine weitere solche Verringerung der Tröpfchengröße beim Austritt an der scharfen Kante des Medienausganges 4, der auch nach außen konisch erweitert sein kann. Es hat sich gezeigt, daß die Tröpfchengröße in dem den Medienausgang 4 verlassenden Kegelstrahl auf bis zu etwa 2 µm oder sogar darunter verringert werden kann und daß größte Tröpfchen höchstens etwa 10 µm messen. Dadurch ergibt sich innerhalb dieser Spanne eine sehr vorteilhafte Größenmischung mit einer Durchschnittsgröße von etwa 5 µm oder weniger.

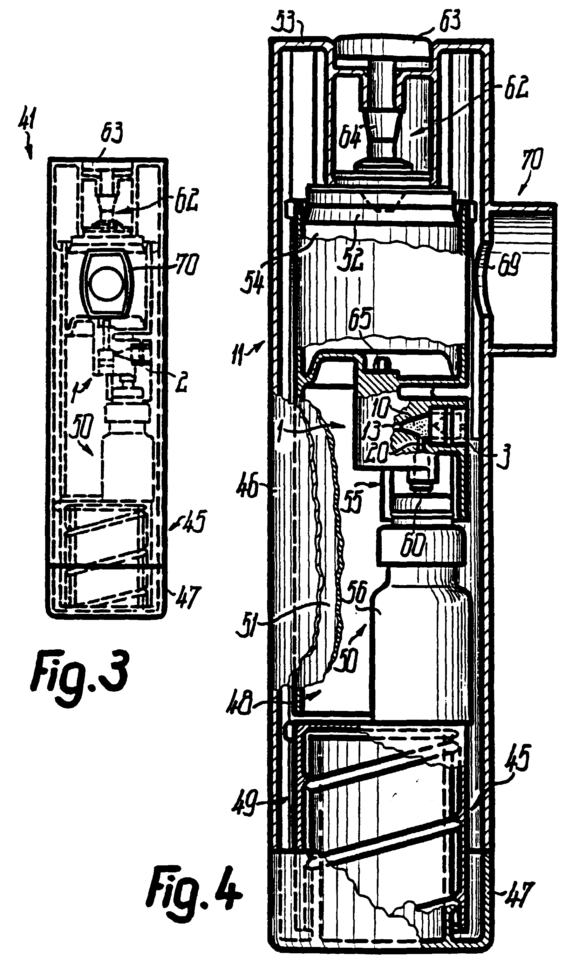

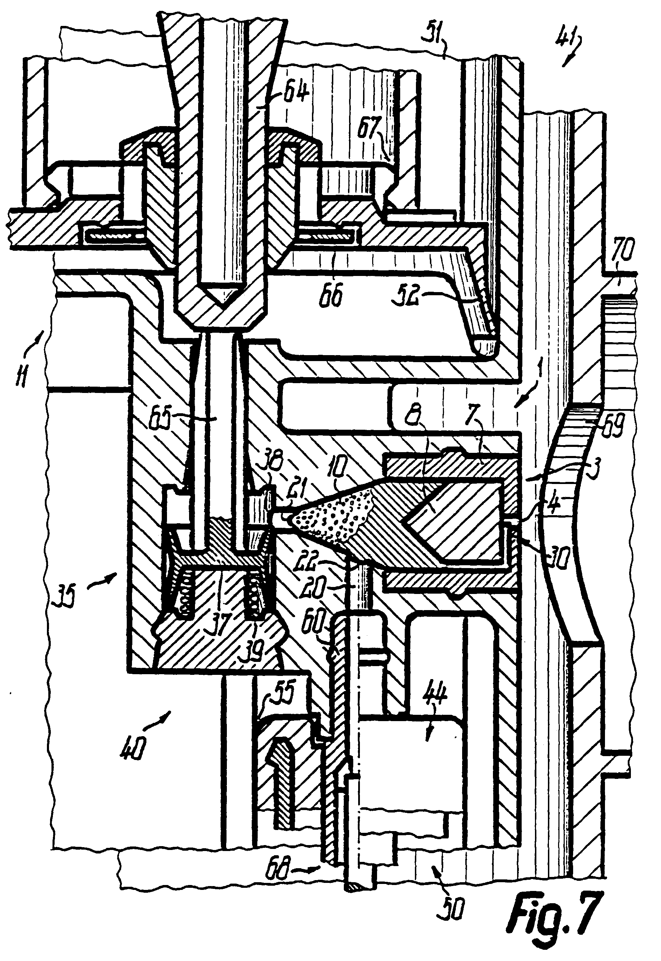

Die Austrageinrichtung 1 arbeitet besonders vorteilhaft, wenn sie zu einer Baueinheit mit einer Austrageinrichtung 41 gemäß den Figuren 3 bis 9 zusammengefaßt ist, welche außer der Fördereinrichtung 11 für Druckluft auch eine oder mehrere Fördereinrichtungen 42 für nicht gasförmiges bzw. flüssiges Medium enthält. Die Fördereinrichtung 11 weist statt und/oder zusätzlich zu einem extern gefüllten Druckgasspeicher eine Luft- bzw. Gaspumpe 43 auf. Entsprechend weist die Fördereinrichtung 42 eine Vorlagepumpe 44 auf. Beide Pumpen sind mit einer Spanneinrichtung 45 zu betätigen und im wesentlichen vollständig verkapselt innerhalb eines langgestreckten Basiskörpers 46 angeordnet, der über seine gesamte Länge durchgehend annähernd konstante Außenquerschnitte haben und nach Art eines Stockgriffes mit einer Hand gegriffen, getragen, betätigt und zum bestimmungsgemäßen Gebrauch eingesetzt werden kann.

In einem Axialabschnitt bzw. an ein Ende des Basiskörpers 46 anschließend, ist eine Handhabe 47 vorgesehen, die praktische eine Fortsetzung des Basiskörpers 46 mit annähernd gleichen Außenquerschnitten bildet, jedoch gegenüber dem Basiskörper 46 wesentlich und gegenüber ihrer Außenweite kürzer ist. Die wie der Basiskörper 46 annähernd zylindrische Handhabe 47 dient als Betätigungsglied der Spanneinrichtung 45 und wirkt zu diesem Zweck auf einen Spanntrieb 49 einer Spanneinheit 48 so, daß die Handhabe 47 in jeder Betätigungsstellung annähernd gleiche Axiallage gegenüber dem Basiskörper 46 einnimmt. Der Spanntrieb weist ein an die Innenseite der Stirnwand der kappenförmigen Handhabe 47 einteilig anschließendes, hülsenförmiges Stellglied mit einem Außengewinde auf, das von einem kappenförmigen Läufer teleskopartig übergriffen ist.

Dieser Läufer greift mit einem Gegenglied bzw. Innengewinde in das Steigungsglied bzw. Gewinde des Stellgliedes ein, verschließt mit seiner Stirnwand das offene Ende des Stellgliedes, ragt wie dieses über die offene Stirnseite des Mantels der Handhabe 47 hinaus und in den Basiskörper 46 hinein, ist axial innerhalb des Basiskörpers 46 verschiebbar sowie gegenüber dem Basiskörper 46 im wesentlichen verdrehgesichert und liegt wie die Handhabe 47 so etwa in der Achse des Basiskörpers 46, daß der Läufer mit seinem Außenmantel bis auf einen ringförmigen Spaltabstand nahe an die etwa gleich weiten Innenseiten der Mäntel des Basiskörpers 46 und der Handhabe 47 reichen kann. Die Gewindeverbindung kann mit degressiver Steigerung z. B. so ausgebildet sein, daß die Untersetzung von der Handhabe 47 zum Läufer mit zunehmender Verdichtung in der Fördereinrichtung 11 zunimmt. Zur Verdrehsicherung weist der Basiskörper 46 an der Innenseite seines Mantels eine oder mehrere Längsnuten auf, die zweckmäßig jeweils durch zwei parallel nebeneinander liegende Axialstege gebildet sind und in die der Läufer jeweils mit mindestens einem Gleitnocken eingreift, der zweckmäßig im Bereich des in Spannrichtung vorderen Endes liegt. Die Stirnwand der Handhabe 47 bildet auch das zugehörige äußerste Ende der gesamten Austrageinrichtung 41.

Die Vorlagepumpe 44 gehört zu einer Spendereinheit 50, die als in sich geschlossene Baueinheit in den Basiskörper 46 eingesetzt bzw. auswechselbar ist. Die Spendereinheit 50 ist an einem wenigstens teilweise hülsenförmigen Spannkörper 51 von etwa gleicher Außenweite wie der Läufer um einen maximalen Pumphub der Vorlagepumpe 44 verschiebbar angeordnet. Der Spannkörper 51 schließt mit einem Ende annähernd an den Läufer des Spanntriebes 49 an, reicht über die gesamte Länge der Spendereinheit 50, über die zugehörige Erstreckung der daran anschließenden Austrageinrichtung 1 und über die Länge der axial daran anschließenden Gaspumpe 43, deren Zylinder 54 er mit dem vom Spanntrieb 49 abgekehrten äußersten Ende bildet. Auch die Innenweite des Spannkörpers 51 bzw. des Zylinders 54 entspricht etwa derjenigen des Läufers und ist damit nur um die notwendigen Wandungsdicken und die Axialführung kleiner als die Innenweite des Basiskörpers 46. Der Spannkörper 51 ist in der anhand des Läufers beschriebenen Weise gegenüber dem Basiskörper 46 axial verschiebbar und verdrehgesichert, wobei die Gleitberührung im wesentlichen nur im Bereich der Längskantenflächen der Axialstege stattfindet und einer geringen Gleitreibung ausgesetzt ist. Die Verdrehsicherungsnocken des Spannkörpers 51 liegen im Bereich von dessen vom Spanntrieb 49 abgekehrten Ende. Der Grundkörper 6 der Austrageinrichtung 1 ist lagefest innerhalb der Hüllfläche des Spannkörpers 51 angeordnet und einteilig mit einer Zwischen- bzw. Stirnwand ausgebildet, die mit Abstand zwischen den Enden des Spannkörpers 51 den Zylinder 54 von demjenigen, im Mantelbereich teilweise offenen Raum trennt, welcher die Spendereinheit 50 aufnimmt.

Von dem vom Spanntrieb 49 abgekehrten, auf voller Zylinderweite offenen Ende des Spannkörpers 51 her greift in den Zylinder 54 ein ringscheiben- bzw. napfförmiger Kolben 52 ein, der an seiner äußeren Stirnseite mit einem hülsenförmigen Kolbenschaft verbunden ist, welcher einteilig von der Innenseite einer End- bzw Stirnwand 53 des Basiskörpers 46 absteht. Die Stirnwand 53 bildet das von der Handhabe 47 abgekehrte Ende der gesamten Austrageinrichtung 41 und ist als ringartiger Boden einteilig mit dem napfförmigen Basiskörper 46 ausgebildet, gegenüber welchem der Kolben 52 stets axial im wesentlichen feststeht.

Etwa achsgleich zum durchgehend gradlinigen sowie zur Austrageinrichtung 41 etwa achsparallelen Medien-Kanal 20 schließt an dessen von der Achse 12 bzw. der Medienvorlage 10 abgekehrtes Ende eine Steck- und Gleitverbindung 55 an, in welcher die Spendereinheit 50 radial annähernd spielfrei gesichert, jedoch axial um einen vorbestimmten Weg hin- und hergehend verschiebbar ist.

Die Spendereinheit 50 weist an ihrem von der Gleitverbindung 55 abgekehrten Ende einen Vorlagespeicher 56 in Form beispielsweise eines zur Austrageinrichtung 41 etwa achsparallelen bzw. in der Achse des Kanales 20 liegenden Fläschchens auf, dessen von der Gleitverbindung 55 abgekehrter Boden durch einen Schleppkolben 57 gebildet ist. Die Vorlagepumpe 44 ragt durch den Hals des Speichers 56 über den größten Teil ihrer Länge in den Speicher 56 und mit ihrem inneren Ende in eine eng an dieses angepaßte napfförmige Vertiefung des Schleppkolbens 57. Die Vorlagepumpe 44 ist z. B. mit einem Krimpring oder dgl. gegen eine äußere Stirnfläche des Halses des Speichers 56 gespannt und so abgedichtet an diesem befestigt. Die Vorlagepumpe 44 ist als Schubkolbenpumpe ausgebildet, weist an ihrem inneren Ende einen Einlaß mit einem als selbstsperrendes Überdruckventil ausgebildeten Einlaßventil 58 auf und enthält eine Kolbeneinheit 68, die eine druck- und/oder wegabhängig öffnendes sowie durch Federbelastung selbstschließendes Auslaßventil 59 bildet. Zu diesen Zweck bildet ein zentraler Schaft der Kolbeneinheit 68 einen ringförmigen Ventilsitz und ein hülsenförmiger Pumpkolben einen axial demgegenüber bewegbaren Ventilkörper sowie in seinem Innern einen Auslaßkanal, durch welchen das Medium aus der Druckkammer der Vorlagepumpe 44 durch den Pumpkolben in einen Stößel 60 austreten kann.

Dieser Stößel 60, der einen im wesentlichen außerhalb des Speichers 56 liegenden Zylinderdeckel des Gehäuses der Vorlagepumpe 44 verschiebbar durchsetzt, ragt mit seinem äußeren Ende in den Grundkörper 6 hinein, mit welchem er über eine axial sichernde Steck-, Klemm- und/oder Schnappverbindung so verbunden ist, daß sein äußeres Ende des Auslaßkanales unmittelbar an das zugehörige Ende des achsgleich liegenden Kanales 20 anschließt. Der Zylinderdeckel bildet mit seinem frei über den Krimpring vorstehenden Außenumfang gleichzeitig das Gleitglied der Gleitverbindung 55. Innerhalb des Gehäuses der Vorlagepumpe 44 ist eine Rückstellfeder vorgesehen, die einerseits die Ventilfeder des Kugel- bzw. Einlaßventiles 58 und andererseits die Rückstellfeder der Kolbeneinheit 68 und damit die Rückstellfeder zur Rückstellung der Spendereinheit 50 gegenüber dem Grundkörper 6 bildet. Die Spendereinheit 50 ist auch im herausgenommenen Zustand als Austragvorrichtung durch Betätigen des Stößels 60 voll funktionsfähig.

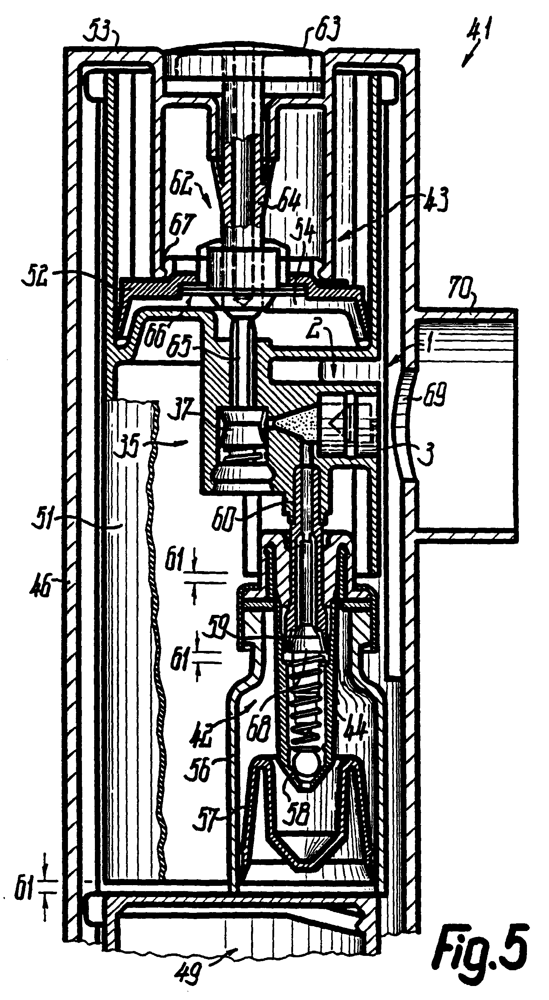

Der Arbeitshub der Vorlagepumpe 44 für den Austrag einer dosierten Charge ist sehr klein und kann z. B. in der Größenordnung eines oder weniger Millimeter liegen. Um diesen Hub 61, der in Fig. 5 für die Bewegung der Spendereinheit 50 gegenüber der Gleitverbindung 55, die Bewegung des Pumpkolbens gegenüber dem Pumpengehäuse und den Leerweg des Läufers des Spanntriebes 49 gegenüber dem Spannkörper 51 dargestellt ist, wird die Spendereinheit 50 durch Betätigen der Spanneinrichtung 45 betätigt. Wird die Handhabe 47 gegenüber dem Basiskörper 46 gedreht, so wird der Läufer des Spanntriebes 49 axial bewegt. Da an der Außenseite der Stirnwand des Läufers das zugehörige Ende des Speichers 56 bzw. der Spendereinheit 50 unter der Kraft von deren Rückstellfeder anliegt und das zugehörige Ende des Spannkörpers 51 in der Ausgangsstellung einen dem Hub 61 entsprechenden Spaltabstand von dieser Stirnwand hat, wird zunächst die Spendereinheit 50 gegenüber dem Spannkörper 51 axial verstellt und dadurch ein Pumphub 61 ausgeführt. Am Ende des Pumphubes schlägt der Pumpkolben mit seinem Ende an einer inneren Ringschulter des Pumpraumes an, wodurch der Pumpkolben vom Ventilsitz abgehoben und das Auslaßventil 59 geöffnet wird. Dadurch wird aus dem Pumpraum und durch den Auslaßkanal ein Tropfen Medium in den Kanal 20 und von dort an den Eingang 22 ausgetragen, wonach sich dieser Tropfen in der beschriebenen Weise innerhalb der Medienvorlage 10 ausbreitet.

Am Ende dieses Pumphubes und im weiteren Verlauf der Betätigung der Handhabe 47 wird nunmehr auch der Spannkörper 51 durch Anschlag mitgenommen, während das Auslaßventil 59 geöffnet bleiben kann. Der nun unmittelbar an die Endfläche des Läufers anschließende Spannkörper 51 wird gegenüber dem Kolben 52 gegen die Kraft des sich im Zylinder 54 aufbauenden Überdruckes aus der Ausgangsstellung gemäß Fig. 4 bis in die Endstellung gemäß Fig. 5 bewegt, in welcher das zugehörige Ende des Spannkörpers 51 nahezu an der Innenseite der Stirnwand 53 anschlägt. Der Läufer des Spanngliedes ragt nun mit dem größten Teil seiner Länge über das Stellglied hinaus. Zur Sicherung dieser Spannlage, auch bei freigegebener Handhabe 47, kann das Gewinde des Spanntriebes 49 selbsthemmend ausgebildet und/oder dem Läufer eine federnde Rastung zugeordnet sein, die durch entsprechend kräftiges Zurückdrehen der Handhabe 47, nicht jedoch durch Axialdruck auf den Spannkörper 51, überwunden werden kann. In der Spannlage befindet sich die Innenseite der Bodenwand des Kolbens 52 mit geringem Abstand von der Zwischenwand des Basiskörpers 46, die napfförmig ausgebildet ist und in den Kolben 52 eingreift.

Um nun die Austrageinrichtungen 1, 41 für einen Austragvorgang ins Freie auszulösen, ist eine Auslöseeinrichtung 62 vorgesehen, die mit einer von der Handhabe 47 gesonderten und im Bereich des anderen Endes der Austrageinrichtung 41 liegenden Handhabe 63 über einen Stößel 64 zu betätigen ist. Die knopf- bzw. scheibenförmige Handhabe 63 liegt im wesentlichen versenkt in einer zentralen Vertiefung der Stirnwand 53 und ist mit ihrem einteiligen Stößel 64 von außen so in eine Gleitführung eingesetzt, daß sie durch ein am Stößel 64 vorgesehenes Schnapp- und Widerhakenglied gegen Rückzug gesichert und in der Ausgangsstellung anschlagbegrenzt ist.

Der Ventilkörper 37 des Ventiles 35 weist einen in der Achse der Austrageinrichtung 41 durch den Grundkörper 6 bzw. die zugehörige Stirnwand in den Zylinder 54 ragenden Ventilschaft 65 auf, der im Querschnitt z. B. kreuzförmig so profiliert ist, daß zwischen seiner Außenfläche und der ihn aufnehmenden Bohrung ein oder mehrere Längskanäle gebildet sind, welche den Zylinder 54 permanent mit der Eingangsseite der Ventilkammer 36 verbinden. Der innere Endabschnitt des Stößels 64 durchsetzt gleit- bzw. längsverschiebbar, jedoch über eine gesondere, verpreßte Hülsendichtung druckdicht abgedichtet und im Abstand innerhalb des zugehörigen Kolbenschaftes die Stirnwand des Kolbens 52 so, daß die Endfläche des Stößels 64 bei Ausgangslage der Handhabe 63 und Spannlage des Spannkörpers 51 wenigstens annähernd an der Endfläche des Ventilschaftes 65 anliegt, das Ventil 35 aber noch geschlossen ist.

Wird nun die Auslöseeinrichtung 62 durch Fingerdruck auf die äußere Stirnfläche der Handhabe 63 gegen die Kraft der Ventilfeder 39 betätigt, so öffnet das Ventil 35 in der beschriebenen Weise und die im Zylinder 54 vorgespannte Druckluft entweicht über die Schaftkanäle und die Ventilkammer 36 zum Eingang 21, um die beschriebenen Funktionen der Austrageinrichtung 1 zu bewirken. Dieser Vorgang ist jederzeit dadurch abzubrechen bzw. zu unterbrechen, daß die Handhabe 63 freigegeben wird, da dann diese, wie auch das Ventil 35, allein durch die Ventilfeder 39 zur Ausgangsstellung zurückbewegt werden. Durch Wahl des Betätigungsschubes kann ggf. auch der Durchlaßquerschnitt des Kammerausganges 38 stufenlos variiert werden. Nach vollständiger Öffnung des Kammerausganges 38 ist der Hubweg durch einen von der Ventilfeder 39 umgebenen Anschlagschaft begrenzt, welcher in den das Ventilgehäuse bildenden Grundkörper 6 auf der vom Ventilschaft 65 abgekehrten Seite des Ventilkörpers 37 eingesprengt ist und ein Widerlager für die Ventilfeder 39 bildet. Dieser Anschlagschaft liegt seitlich unmittelbar benachbart zum Stößel 60, welcher einschließlich der Spendereinheit 50 derart seitlich gegenüber der Ventil- bzw. Mittelachse der Austrageinrichtung 41 versetzt ist, daß er zwischen dieser Mittelachse und dem Medienausgang 4 liegt.

Ein Einlaßventil 66 der Gaspumpe 43 bildet eine Baueinheit mit dem Kolben 52, welcher an der Innenseite seiner Stirnwand einen Ventilsitz für einen ringscheibenförmigen und vom Stößel 64 durchsetzten Ventilkörper bildet. Die vormontierte Baueinheit aus Kolben 52 und Einlaßventil 66 ist über eine axiale Schnappverbindung 67 am zugehörigen Kolbenschaft befestigt. Nach Durchführung eines Austrages kann der Spanntrieb 49 durch Zurückdrehen der Handhabe 47 wieder in seine Ausgangslage zurückbewegt werden. Für die entsprechende Rückstellung des Spannkörpers 51 kann eine formschlüssige Verbindung zwischen dem Läufer des Spanntriebes 49 und dem Spannkörper 51, eine im Zylinder 54 liegende Druckfeder und/oder eine an der Außenseite des Spannkörpers 51 liegende Rückstellfeder vorgesehen sein, so daß der Spannkörper 51 mit dem Läufer des Spanntriebes 49 zur Ausgangsstellung zurückläuft. Die Vorlagepumpe 44 bzw. deren Auslaßventil 59 werden durch die zugehörige Rückstellfeder zur Ausgangslage zurückgestellt.

Bei der Rückstellbewegung öffnet das Einlaßventil 58 und es wird unter selbsttätigem Nachrücken des Schleppkolbens 57 aus dem Speicher 56 eine weitere Mediencharge in den Pumpraum der Vorlagepumpe 44 angesaugt. Außerdem wird durch selbsttätiges Öffnen des als Rückschlagventil ausgebildeten Einlaßventiles 66 in den Zylinder 44 Luft von außen angesaugt, die dabei außerhalb des Stößels 64 und der zugehörigen Dichtung die Stirnwand des Kolbens 52, den zugehörigen Kolbenschaft und die Lageröffnung für den Stößel 64 in der Stirnwand 53 durchströmt. Schließlich schließt zu Beginn der Rückstellbewegung das Ventil 35 selbsttätig. Nach derartiger Einstellung des Ausgangszustandes kann ein weiterer Austragzyklus in der beschriebenen Weise durchgeführt werden.

Da die Austrageinrichtung 1 unmittelbar benachbart zur Druckkammer bzw. zum Zylinder 54 liegt, sind die Leitungswege zwischen dieser Druckkammer und dem Eingang 21 sehr kurz, so daß sich sehr geringe Verluste in der Strömungsenergie ergeben. Aufgrund der beschriebenen Ausbildung befindet sich der Medienausgang 4 in der genannten Ausgangstellung in einer anderen Axiallage gegenüber dem Basiskörper 46 als in der Spannlage. In der Spannlage liegt der Medienausgang 4 annähernd achsgleich zu einem annähernd flach-ovalen Austragstutzen 70, der einteilig mit dem Mantel des Basiskörpers 46 ausgebildet ist und über dessen Außenseite frei vorsteht. Etwa in der Achse des Austragstutzens 70 ist der Mantel des Basiskörpers 46 mit einem Durchbruch bzw. Durchgang 69 versehen, der gegenüber der größten Weite des Austragstutzens 70 kleinere Weite hat, jedoch so weit ist, daß er den aus der Austragdüse 3 austretenden Düsenstrahl praktisch nicht beeinfluß bzw. von diesem Strahl an seinen Begrenzungen nicht berührt wird. Die größere Querschnittserstreckung des Austragstutzens 70 liegt etwa parallel zur Längsrichtung der Austrageinrichtung 41, so daß diese etwa parallel zum Mund auszurichten ist, wenn der Austragstutzen 70 mit den Lippen umschlossen wird. In der Ausgangsstellung ist die Austragdüse 3 demgegenüber axial versetzt, d. h. daß sie im Abstand vom Austragstutzen 70 vollständig verdeckt und abgeschirmt innerhalb des Basiskörpers 46 liegt und gegen Verschmutzungen geschützt ist. Der Durchgang 69 ist dann im wesentlichen durch den Mantel des Spannkörpers 51 verschlossen. In Fig. 3 ist der Darstellung halber der Austragstutzen 70 um 90° versetzt gegenüber dem Austragkopf 2 dargestellt.

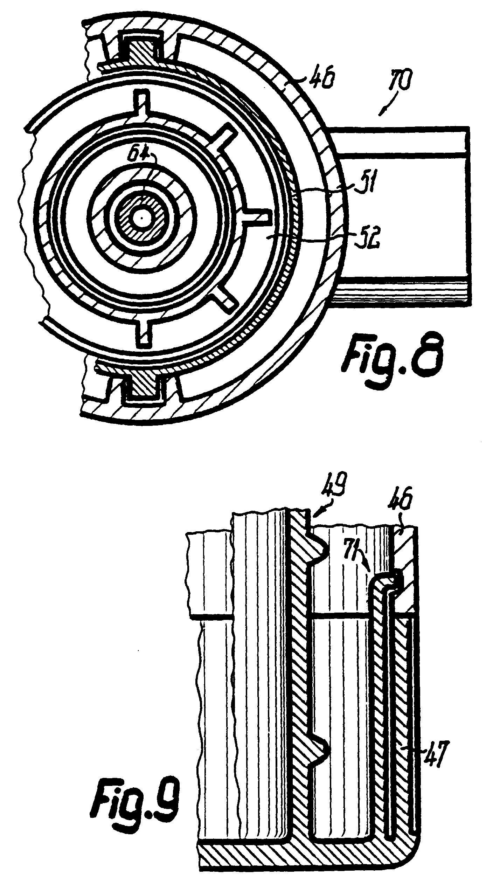

Wie insbesondere Fig. 8 zeigt, ist der Kolbenschaft des Kolbens 52 an Außenumfang durch radial vorstehende Längsrippen versteift, von denen in Fig. 8 der Einfachheit halber nur ein Teil dargestellt ist und die zur sicheren Druckabstützung bis an die rückwärtige Stirnfläche des elastisch rückfedernd verformbaren Kolbens 52 reichen.

Fig. 9 zeigt eine Axialsicherung 71 für die Handhabe 47, die gleichzeitig einen End- bzw. Abschlußdeckel für das zugehörige Ende des Basiskörpers 46 bildet. Die Axialsicherung 71 kann beispielsweise eine radial federnde Innenhülse aufweisen, die mit geringen radialen Spaltabstand vom Innenumfang des Mantels der Handhabe 47 liegt, einteilig von der Innenseite von deren Stirnwand ausgeht, über die offene Kappenstirnseite weniger weit als das innerhalb ihr liegende Stellglied des Spanntriebes 49 hinausragt und am freien Ende einen radial nah außen gerichteten Ringflansch bildet, welcher in eine Ringnut am Innenumfang des Basiskörpers 46 eingreift. Dadurch ist eine lediglich durch axiales Zusammensetzen selbst einrückende Schnappverbindung gebildet, die ggf. so ausgebildet sein kann, daß sie durch Aufbringen einer entsprechend großen axialen Abzugkraft von selbst ausrastet und so ein zerstörungsfreies Öffnen des Basiskörpers 46 ermöglicht.

Zur Montage kann zunächst von diesem offenen Ende des Basiskörpers 46 her der Kolben 52 eingesetzt und durch die axial anschlagende Schnappverbindung 67 gesichert werden. Danach kann die vormontierte Baueinheit aus Spannkörper 51, Spendereinheit 50 und Austrageinheit 1 von dem offenen Ende her in den Basiskörper 46 eingeschoben werden. Es ist auch denkbar, den bereits im Zylinder 54 befindlichen Kolben 52 gemeinsam mit dieser Baueinheit einzusetzen und einzuschnappen. Vor oder nach der Montage des Kolbens 52, bzw. der Baueinheit, kann die Handhabe 63 mit dem Stößel 64 vom anderen Ende und in entgegengesetzter Richtung eingesetzt sowie eingeschnappt werden. Wiederum vor oder nach dem Einsetzen der Handhabe 63 kann die Handhabe 47 mit dem vormontierten Spanntrieb 49 montiert werden, der geeignet ist, durch Betätigung den noch im Zylinder 54 befindlichen Kolben 52 zur Herstellung der Schnappverbindung 67 einzurasten. Nach Lösen der Handhabe 47 kann die Spendereinheit 50 axial aus der Steckverbindung 55 zerstörungsfrei herausgezogen und gegen eine andere ausgewechselt werden.

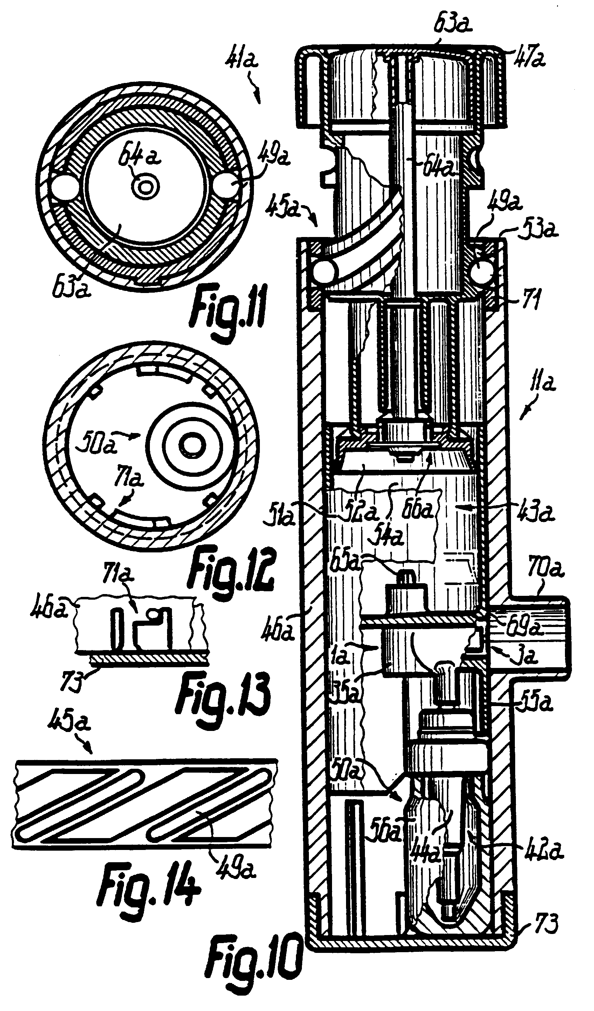

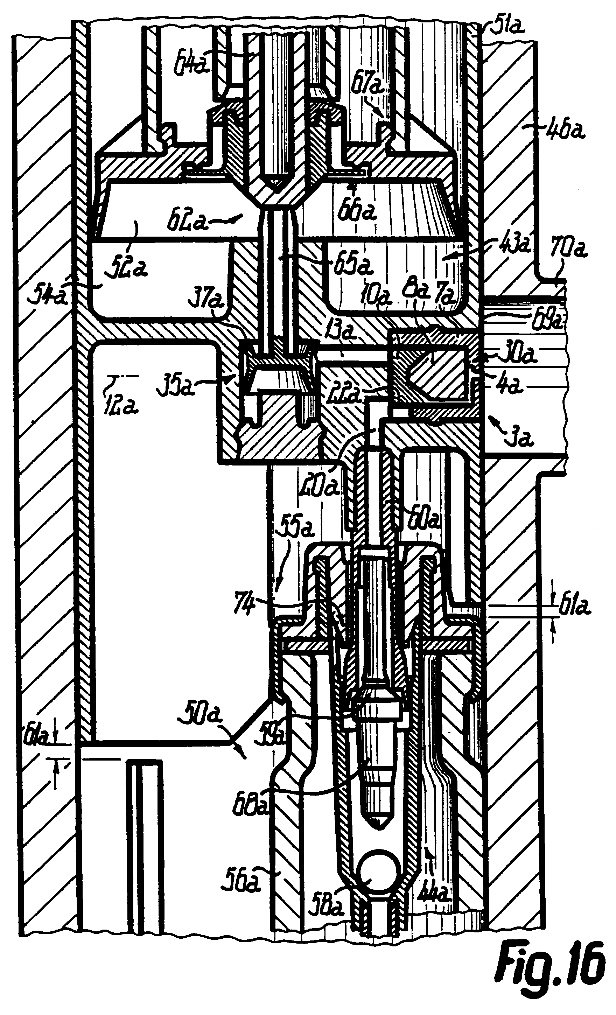

In den Figuren 10 bis 16 sind für einander entsprechende Teile die gleichen Bezugszeichen wie in den Figuren 1 bis 9, jedoch mit dem Index "a" verwendet, weshalb alle Bescheibungsteile sinngemäß für alle Ausführungsformen gelten. Die Austrageinrichtungen 1, 1a bzw. 41, 41a können in den Ausführungsformen auch gegeneinander ausgetauscht oder jeweils bei einer einzigen Ausführungsform vorgesehen sein, um z. B. unterschiedliche Medien aus unterschiedlichen Medienausgängen auszutragen bzw. zwei oder mehr Spanneinrichtungen 45, 45a zur Vorspannung eines einzigen oder mehrerer Druckspeicher einzusetzen.

Die Austrageinrichtung 1a weist hier eine Medienvorlage 10a auf, die im wesentlichen mit dem hinteren Ende der Düsenkappe 7a bündig abschließt und in die der Eingang des Kanales 13a exzentrisch zur Achse 12a so mündet, daß er auf der vom Medien-Eingang 22a abgekehrten Seite der Achse 12a liegt, sowie annähernd an den Innenumfang des Mantels der Düsenkappe 7a angrenzt. Der Medien-Kanal 20a mündet an der hinteren, annähernd ebenen Stirnfläche der Medienvorlage 10a und/oder an einen daran anschließenden Umfangsbereich, wofür er den Mantel der Düsenkappe 7a radial durchsetzt. Im Falle der Austrageinrichtung 1 ist die Medienvorlage 10 länger als der Kernkörper 8 und im Falle der Austrageinrichtung 1a kürzer, so daß sich ein annähernd ringscheiben- und napfförmiger Füllkörper ergibt.

Beide Handhaben 47a, 63a liegen hier am selben Ende 53a des Basiskörpers 46a, etwa koaxial ineinander und sind von diesem Ende 53 her eingesetzt, das auf voller übriger Innenweite des Basiskörpers 46a offen ist. Die Handhabe 47a weist einen von der Innenseite ihrer Stirnwand einteilig vorstehenden, hülsenförmigen Schaft auf, der am Außenumfang mit einer innengewindeartigen Steigung versehen ist und im Anschluß an diese in eine Verlängerung übergeht, welche den Kolbenschaft für den Kolben 52a bildet. Innerhalb der im Querschnitt doppelt ringförmigen Handhabe 47a liegt die kappenförmige Handhabe 63a, die im Bereich der Innenseite ihrer Stirnwand über eine Schnappverbindung mit dem gesonderten Stößel 64a verbunden ist. In einem im Inneren erweiterten Endabschnitt des offenen Endes 53a und annähernd an dieses anschließend ist ein hülsenförmiger Käfig 71 eingesetzt, der eine der Anzahl der Gänge des Innengewindes entsprechende Anzahl kugelförmiger Übertragungs- bzw. Wälzkörper trägt, die gleichmäßig über den Umfang verteilt sind und in das Innengewinde eingreifen. Der Spanntrieb 49a kann dadurch als vormontierte Bauteinheit gemeinsam mit der Handhabe 63a, dem Stößel 64a, dem Kolben 52a mit Einlaßventil 66a und dem Käfig 71 mit Übertragungsgliedern am Basiskörper 46a montiert werden.

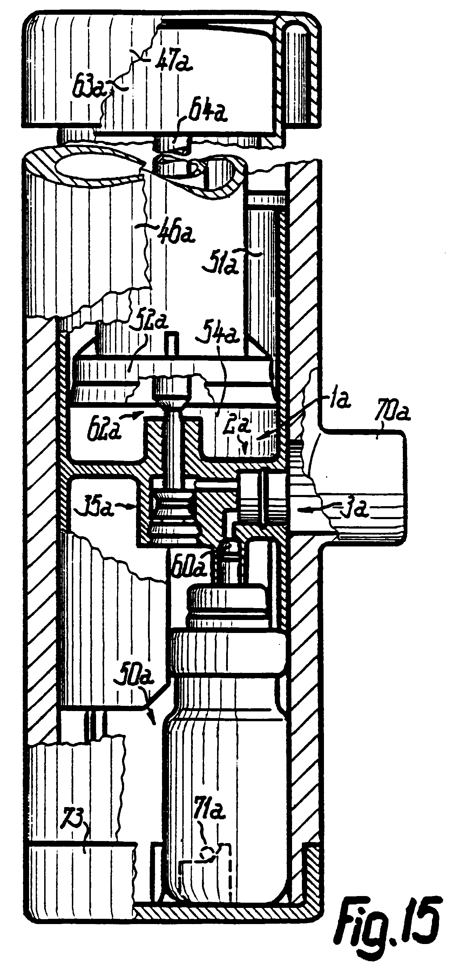

Da in diesem Fall die Handhaben 47a, 63a und der Kolben 52a die axiale Spannbewegung der Spanneinrichtung 45a gegenüber dem Basiskörper 46a ausführen, führt der Stellkörper 51a im wesentlichen nur den Hub 61a der Fördereinrichtung 42a aus. Dieser Hub 61a erfolgt dabei erst, wenn nach einem entsprechend großen Hubweg des Kolbens 52a der Druck im Zylinder 54a so groß ist, daß aufgrund der entsprechenden pneumatischen Kraftverbindung die Betätigungskraft erreicht ist, welche zur Betätigung der Vorlagepumpe 44a benötigt wird. Zur Sicherung der Spanneinrichtung 45a im Spannzustand, in welchem die Handhabe 47a das offene Ende 53a annähernd dicht schließt, kann eine entsprechende federnde Rastung vorgesehen sein. Z. B. können die, eine Steigerung von etwa 30° aufweisenden und in Figur 14 als Abwicklung dargestellten Steigungsnuten benachbart zu ihren zugehörigen Enden Rastnocken zum Überspringen durch die Übertragungsglieder in beiden Bewegungsrichtungen aufweisen, falls der Handhabe 47a eine entsprechend große Betätigungskraft vermittelt wird.

Bei dieser Ausführungsform kann die Austrageinrichtung 1a bzw. die Spendereinheit 50a gegenüber dem Basiskörper 46a zumindest axial im wesentlichen feststehend angeordnet sein und/oder vom offenen Ende 53a her, ggf. als vormontierte Baueinheit, montiert werden. Das andere Ende des Basiskörpers 46a ist jedoch ebenfalls auf der genannten vollen Innenweite offen und mit einem kappenförmigen Deckel 73 verschlossen, dessen formschlüssige Axialsicherung 71a durch eine Verriegelung gebildet ist, welche durch entgegengesetzt gerichtetes Drehen des Deckels 73 gegenüber dem Basiskörper 46a verriegelt bzw. gelöst werden kann. Der Speicher 56a der Spendereinheit 50 reicht mit seinem Boden annähernd bis an die Innenseite der Stirnwand des Deckels 73, gegenüber welchem die Spendereinheit 50a gegen die axiale Betätigungskraft beim Hub 61a abgestützt ist. Nach zerstörungsfreiem Abnehmen des Deckels 73 ist auch von diesem Ende her Zugänglichkeit zum Einsetzen bzw. Herausnehmen der Spendereinheit 50a gegeben.

Das Ende des Hubes 61a kann auch durch Anschlag des Stellkörpers 51a am Basiskörper 46a, z. B. an einer Innenrippe, begrenzt sein. Die Gleitführung 55a kann des weiteren mit einem größeren bzw. größten Außenumfang der Spendereinheit 50a als Gleitfläche zusammenwirken, nämlich mit dem Befestigungs- bzw. Krimpring für die Vorlagepumpe 44a und/oder mit dem Außenumfang des Speichers 56a.

Der Speicher 56a ist in diesem Fall durch ein am Boden feststehend und einteilig geschlossenes Fläschchen aus Glas oder dgl. gebildet, bis in dessen zu einem Bodensumpf verengten Bodenbereich die Vorlagepumpe 44a mit dem Einlaß eines Saug- bzw. Steigrohres reicht, das die Leitungsverbindung zum Einlaßventil 58a herstellt. Die Spendereinheit 50a bildet dadurch auch im nichtmontierten Zustand ein nach außen durch das vorgespannte Auslaßventil 59a druckdicht verschlossenes System, bei dem eine Verkeimung des gespeicherten Mediums auch nach langer Lagerzeit nicht zu befürchten ist. Ein für den Speicherraum vorgesehenes Belüftungsventil 74, dessen beide Ventilschließteile zweckmäßig einerseits durch den Pumpkolben und andererseits durch den Zylinderdeckel gebildet sind, ist in diesem Ausgangszustand ebenfalls unter Vorspannung druckdicht geschlossen.

Der ringförmige Ventilteller des Einlaßventiles 66a der Gaspumpe 43a kann auch einteilig mit der Dichtmuffe ausgebildet sein, durch welche der Stößel 64a im Bereich des Kolbens 52a abgedichtet hindurchgeführt ist. Der Ventilteller steht in diesem Fall über den Außenumfang der Dichtmuffe ringscheibenförmig vor und ist für die Ventilöffnung durch Biegung in sich rückfedernd elastisch verformbar. Wie insbesondere Figur 1 zeigt, kann der Innenumfang der Ventilkammer 36 auch so abgestuft sein, daß der Kammerausgang 38 in einem sich erweiternden und/oder erweiterten Abschnitt liegt, so daß hier eine Art innerer Ringschulter gebildet ist. In Schließlage liegt der Ventilkörper 37 am engeren Innenumfang, den er bei Betätigung mit seiner dem Kammerausgang 38 gegen den Druck im Zylinder 54 sperrenden Dichtlippe schlagartig so verläßt, daß der Durchfluß-Verbindungsquerschnitt zum Kammerausgang 38 nicht allmählich vergrößert, sondern schlagartig auf maximale Weite freigegeben ist, weil die Dichtlippe des Ventilkörpers 37 dann nicht mehr am Innenumfang der Ventilkammer 36 anliegt.

## Patentansprüche

1. Austrageinrichtung für fließfähige Medien, insbesondere für manuell betätigbare Austragvorrichtungen (41, 41a), mit einem Grundkörper (6), einem Medienausgang (4, 4a) und einer Verteileinrichtung (10, 10a) zur Ausbreitung des Mediums, dadurch gekennzeichnet, daß der Verteileinrichtung mindestens eine Medienvorlage (10, 10a) zur räumlich ausgebreiteten Bereitstellung fein verteilten Mediums nahezu in einem Ruhezustand zugeordnet ist, und daß Druckmittel (11, 11a) vorgesehen sind, um das in der Bereitstellung befindliche Medium einer nochmals beginnenden Strömung hin zum Medienausgang (4, 4a) auszusetzen.

2. Austrageinrichtung nach Anspruch 1, dadurch gekennzeichnet, daß sie mindestens einen Austragkopf (2, 2a) aufweist, an dem der nach außen mündende Medienausgang (4, 4a) vorgesehen ist, welchem mindestens eine Verteileinrichtung (10, 10a) zur Ausbreitung wenigstens eines Mediums auf eine zunehmende Ausdehnung durch eine Fördereinrichtung zugeordnet ist, wobei vorzugsweise wenigstens eine Verteileinrichtung mindestens eine Medienvorlage (10, 10a) zur verteilten und stehenden Aufnahme wenigstens eines Mediums aufweist und insbesondere mindestens eine Medienvorlage (10) im Strömungsweg der, wenigstens eine Druckstoß-Fördereinnichtung (11, 11a) umfassenden, Druckmittel zur Ablösung und Mitnahme mindestens eines Mediums von wenigstens einer Medienvorlage (10, 10a) liegt und/oder wobei wenigstens eine Verteileinrichtung (10, 10a) zur räumlichen Ausbreitung des Mediums auf ein zunehmendes Raumvolumen ausgebildet ist, insbesondere eine Verteileinrichtung innerhalb einer Außenbegrenzung wenigstens einer Medienvorlage (10, 10a) mindestens eine zergliederte Oberflächenstruktur zur haftenden Benetzung mit wenigstens einem Medium aufweist und vorzugsweise mindestens eine Oberflächenstruktur durch wenigstens einen offenporig porösen Vorlagekörper (9) gebildet ist.

3. Austrageinrichtung nach Anspruch 1 oder 2, dadurch gekennzeichnet, daß wenigstens eine Medienvorlage (10, 10a) eine vernetzte Faser- und/oder Membramstruktur aufweist, daß insbesondere eine Medienvorlage (10, 10a) schwammartig elastisch kompressibel und/oder unter Vorspannung zusammengedrückt ist und daß vorzugsweise eine Medienvorlage (10, 10a) wenigstens teilweise aus Schaumstoff unterschiedlicher Porenweite besteht und/oder ein innerhalb ihrer Peripherieflächen im wesentlichen durchgehender Vollkörper ist.

4. Austrageinrichtung nach einem der vorhergehenden Ansprüche, dadurch gekennzeichnet, daß mindestens eine Medienvorlage (10, 10a) wenigstens teilweise im Bereich einer äußeren Begrenzung (17, 18, 19) liegt und/oder an dieser anliegt, daß insbesondere eine Medienvorlage (10, 10a) wenigstens teilweise innerhalb eines Gehäuses liegt und daß vorzugsweise eine Medienvorlage (10, 10a) im wesentlichen vollständig umschlossen angeordnet sowie nur im Bereich mindestens eines Medienanschlusses (21, 22) frei liegt und/oder zwischen ihren Enden an einen radialen Medienanschluß (22) angrenzt.

5. Austrageinrichtung nach einem der vorhergehenden Ansprüche, dadurch gekennzeichnet, daß wenigstens eine Medienvorlage (10, 10a) in einer Strömungsrichtung zunehmende und/oder abnehmende Ausdehnung aufweist, daß insbesondere eine Medienvorlage (10, 10a) im Anschluß an mindestens einen Eingang (21, 22) und in Durchströmungsrichtung einer Fördereinrichtung (11, 11a) im Querschnitt annähernd stetig zunimmt, und daß vorzugsweise eine Medienvorlage (10, 10a) anschließend an und annähernd bis zu wenigstens einem Vorlagenausgang (24) im Querschnitt annähernd stetig abnimmt und/oder einen Abschnitt (15) mit etwa konstanten Querschnitten aufweist.

6. Austrageinrichtung nach einem der vorhergehenden Ansprüche, dadurch gekennzeichnet, daß wenigstens eine gegen mindestens eine Medienvorlage (10) gerichtete Druckmündung (38) in einem Bereich annähernd kleinsten Querschnittes der Medienvorlage (10) und/oder zu dieser etwa achsgleich liegt, daß insbesondere eine von einer stirnseitigen Druckmündung (38) abgekehrte Seite und/oder ein Umfang einer Medienvorlage (10, 10a) im wesentlichen vollständig an eine druckfest harte Begrenzung (19) angrenzt, und daß vorzugsweise wenigstens ein Vorlagenausgang (24) im Bereich eines Umfanges einer Medienvorlage (10, 10a) liegt und/oder eine Druckmündung (38) etwa gleichen Austrittsquerschnitt wie der zugehörige kleinste Querschnitt einer Medienvorlage (10) hat.

7. Austrageinrichtung nach einen der vorhergehenden Ansprüche, dadurch gekennzeichnet, daß wenigstens eine Medienvorlage (10, 10a) wenigstens teilweise Hohlquerschnitte aufweist, daß insbesondere eine Medienvorlage (10, 10a) an einer von einer Druckmündung (38) entfernten Seite wenigstens eine annähernd über deren gesamte Weite reichenden Vertiefung aufweist, und daß vorzugsweise eine Medienvorlage (10, 10a) an einer von einer Druckmündung (38) entfernten Seite an eine zu wenigstens einem Vorlagen-Ausgang (24) führende Leitfläche (34) angrenzt und/oder mit einem eine Vertiefung eindrückenden Vorsprung in ihrer Struktur verdichtet ist.

8. Austrageinrichtung nach einem der vorhergehenden Ansprüche, dadurch gekennzeichnet, daß wenigsten einer Medienvorlage (10, 10a) mindestens ein Prallzerstäuber (30, 30a) nachgeschaltet ist, daß insbesondere an wenigstens einen Vorlagen-Ausgang (24) einer Medienvorlage (10, 10a) ein Düsenkanal (23) anschließt, der mindestens eine zu wenigstens einem Prallzerstäuber (30, 30a) führende Kanal-Umlenkung (28) aufweist, und daß vorzugsweise eine Prallfläche (31) eines Prallzerstäubers (30, 30a) quer zur abließenden Strömungsrichtung und/oder eine Prallfläche (31) unmittelbar vor einem den Medienausgang (4, 4a) bildenden Düsen-Endkanal (5) liegt, der gegenüber der Querschnittsfläche des Kanales (28) kleinere Durchlaßquerschnitte aufweist.

9. Austrageinrichtung nach einem der vorhergehenden Ansprüche, dadurch gekennzeichnet, daß mindestens ein Medienausgang (4, 4a) mit wenigstens einer Medienvorlage (10, 10a) mindestens eine Düseneinheit bildet, daß insbesondere in einer von einem Medienausgang (4, 4a) durchsetzten Düsenkappe (7, 7a) wenigstens ein Kernkörper (8, 8a) angeordnet ist, und daß vorzugsweise ein näher beim Medienausgang (4, 4a) liegender sowie druckfester Kernkörper (8, 8a) mit wenigstens einer Stirnfläche eine Leit- bzw. Prallfläche (34, 31) bildet und/oder ein über ein hinteres Ende der Düsenkappe (7, 7a) vorstehender Kernkörper als Medienvorlage (10, 10a) ausgebildet ist.

10. Austrageinrichtung, nach einem der vorhergehenden Ansprüche, dadurch gekennzeichnet, daß wenigstens eine Fördereinrichtung (11, 42,) mindestens eine Spenderquelle aufweist, die eine bauliche Einheit mit wenigstens einem Austragkopf (2, 2a) bildet, daß insbesondere mindestens eine Spenderquelle als Gaspumpe (43) und/oder wenigstens eine Spenderquelle als Vorlagepumpe (44) für ein Wirkmedium ausgebildet ist, und daß vorzugsweise eine Vorlagequelle (44) zur Medienalangerung an eine Medienvorlage (10, 10a) und eine Druckstoß-Spenderquelle (43) zur schlagartig beginnenden und im wesentlichen kontinuierlich weiter, sowie durchströmenden Fluidbeaufschlagung des wenigstens teilweise eingelagerten Mediums an die Mediumvorlage (10, 10a) angeschlossen ist.

11. Austrageinrichtung nach Anspruch 10, dadurch gekennzeichnet, daß mindestens eine Druckstoß-Spenderquelle (43) eine Spanneinrichtung (45) zur manuellen Vorspannung und Speicherung einer Druckstoß-Strömungsenergie aufweist, daß insbesondere eine Druckstoß-Spenderquelle (43) einen Druckgas-Speicher (54) aufweist, und daß vorzugsweise Steuermittel (40) zur im wesentlichen zwangsläufigen Voranfolge einer Abgabe einer annähernd vollständigen Austragdosis an eine Medienvorlage (10, 10a) durch eine Vorlage-Spenderquelle (44) vor einem Druckstoß durch eine Druckstoß-Spenderquelle (43) vorgesehen sind.

12. Austrageinrichtung nach einem der vorhergehenden Ansprüche, dadurch gekennzeichnet, daß wenigstens eine Fördereinrichtung (11) über eine Schraubsteigung betätigbar ist, daß insbesondere wenigstens eine Fördereinrichtung (11) durch Drehen einer Handhabe (47, 47a) betätigbar ist, und daß vorzugsweise eine Druckstoß-Spenderquelle (43, 43a) über wenigstens eine Spann-Handhabe (47, 47a) vorspannbar und/oder eine Vorlage-Spenderquelle (44) unter Zwischenschaltung einer Federdämpfung über einen Austraghub betätigbar ist.

13. Austrageinrichtung nach einem der Ansprüche 10 bis 12, dadurch gekennzeichnet, daß ein und derselbe manuelle Betätigungshub einerseits zur Vorspannung wenigstens einer Druckstoß-Spenderquelle (43) und andererseits wenigstens teilweise simultan zur Medieneinlagerung in wenigstens einer Medienvorlage (10, 10a) vorgesehen ist, daß insbesondere eine lineare Hubbewegung eines Hubantriebes eine Druckkammer (54) einer Druckstoß-Spenderquelle (43) unter Druckerhöhung verengt und in den Kraftfluß des Hubantriebes ein Austragantrieb für eine Vorlage-Spenderquelle (44) zwischengeschaltet ist, und daß vorzugsweise in einem Zwischenglied (51, 51a) des Austragantriebes wenigstens ein Austragkopf (2, 2a) angeordnet ist.

14. Austrageinrichtung nach einem der Ansprüche 10 bis 13, dadurch gekennzeichnet, daß wenigstens eine Druckgas-Spenderquelle (43) zur schlagartig beginnenden Druckabgabe aus einem selbstgesicherten Ruhezustand mit wenigstens einer Auslöse-Handhabe (63, 63a) betätigbar ist, daß insbesondere eine Auslöse-Handhabe (63, 63a) als Fingertaste ausgebildet ist, und daß vorzugsweise eine Auslöse-Handhabe (63, 63a) im wesentlichen versenkt in einer Vertiefung und/oder etwa in einer Achse einer Spann-Handhabe (47, 47a) liegt.

15. Austrageinrichtung nach einem der Ansprüche 10 bis 14, dadurch gekennzeichnet, daß wenigstens eine Druckgas-Spenderquelle (43) über mindestens ein Druck-Auslaßventil (35, 35a) sperrbar ist, daß insbesondere wenigstens ein Ventil-Kammerausgang (38) eines Auslaßventiles (35) unmittelbar benachbart zu einem Vorlagen-Eingang (21) einer Medienvorlage (10, 10a) liegt, und daß vorzugsweise ein Ventil-Kammerausgang (38) etwa radial zur zugehörigen Ventilkammer (36) und/oder etwa parallel zu einer Medienvorlage (10, 10a) liegt.

16. Austrageeinrichtung nach einem der Ansprüche 10 bis 15, dadurch gekennzeichnet, daß wenigstens eine Vorlage-Spenderquelle (50) durch eine von mindestens einer funktionsfähigen Druckgas-Spenderquelle (11) gesonderte und zerstörungsfrei leicht trennbare Medium-Baueinheit gebildet ist, daß insbesondere eine Vorlage-Spenderquelle (50) eine Medium-Baueinheit aus selbstansaugender Pumpe (44) und Medienspeicher (56) bildet, und daß vorzugsweise ein Betätigungsstößel (60, 60a) einer Medien-Baueinheit über eine Steckverbindung an einen Austragkopf (2 bzw. 2a) einer Druckstoß-Baueinheit (11 bzw. 11a) anschließbar ist und/oder ein Austragkopf (2 bzw. 2a) zwischen Druckkammern einer Druckgas-Spenderquelle (11) und einer Medien-Spenderquelle (50) liegt.

17. Austrageinrichtung nach einem der Ansprüche 10 bis 16, dadurch gekennzeichnet, daß wenigstens eine Druckgas-Spenderquelle (11) und mindestens eine Vorlage-Spenderquelle (50) im wesentlichen vollständig umschlossen in einem Gehäuseraum eines Außengehäuses (46) angeordnet sind, daß insbesondere auch mindestens ein Austragkopf (2 bzw. 2a) im wesentlichen innerhalb des Gehäuseraumes angeordnet ist, und daß vorzugsweise ein Längsabschnitt des Außengehäuses eine Spann-Handhabe (47) und/oder einen Gehäusedeckel (73) für eine Gehäuseöffnung zum Einsetzen einer Medien-Baueinheit (50 bzw. 50a) bildet.

## Claims

1. Discharge apparatus for flowable media, particularly for manually operable discharge means (41, 41a) having a body (6), a medium outlet (4, 4a) and a distributing device (10, 10a) for spreading the medium, characterized in that with the distributing device is associated at least one medium receiver (10, 10a) for the spatially spread provision of finely dispersed medium virtually in a rest state, and that pressure means (11, 11a) are provided in order to expose the medium in the readiness position to an again starting flow to the medium outlet (4, 4a).

2. Discharge apparatus according to claim 1, characterized in that it has at least one discharge head (2, 2a) on which at least one medium outlet (4, 4a) is outwardly provided, which has associated with it at least one distributing device (10, 10a) for spreading at least one medium to an increasing extension through a conveying device, preferably at least one distributing device has at least one medium receiver (10, 10a) for the distributed and standing reception of at least one medium and in particular at least one medium receiver (10) is located in the flow path of at least one pressure surge conveying device (11, 11a) for the detachment and entraining of at least one medium from at least one medium receiver (10, 10a) and/or at least one distributing device (10, 10a) is constructed for the spatial spreading of the medium to an increasing space volume, particularly a distributing device within an outer boundary of at least one medium receiver (10, 10a) has at least one divided up surface structure for adhesive wetting with at least one medium and preferably at least one surface structure is formed by at least one open-pore, porous receiver body (9).

3. Discharge apparatus according to claim 1 or 2, characterized in that at least one medium receiver (10, 10a) has a crosslinked fibre and/or membrane structure, that in particular one medium receiver (10, 10a) is sponge-like, elastically compressible and/or compressed under pretension and that preferably a medium receiver (10, 10a) comprises at least partly a foam with a different pore width and/or within its peripheral surfaces is substantially a through solid body.

4. Discharge apparatus according to one of the preceding claims, characterized in that at least one medium receiver (10, 10a) is at least partly located in the vicinity of an outer boundary (17, 18, 19) and/or engages thereon, that in particular a medium receiver (10, 10a) is located at least partly within a casing and that preferably a medium receiver (10, 10a) is arranged in substantially completely enclosed manner and only in the vicinity of at least one medium connection (21, 22) is free and/or is adjacent to a radial medium connection (22) between its ends.

5. Discharge apparatus according to one of the preceding claims, characterized in that at least one medium receiver (10, 10a) has in one flow direction an increasing and/or decreasing extension, that in particular a medium receiver (10, 10a) following onto at least one inlet (21, 22) and in the flow direction of a delivery device (11, 11a) cross-sectionally approximately continuously increases and that preferably a medium receiver (10, 10a) following onto and approximately up to at least one receiver outlet (24) cross-sectionally approximately continuously decreases and/or has a portion (15) with approximately constant cross-sections.

6. Discharge apparatus according to one of the preceding claims, characterized in that at least one pressure mouthpiece (38) directed against at least one medium receiver (10) is located in an area of approximately minimum cross-section of the medium receiver (10) and/or is approximately equiaxial thereto, that in particular a side remote from the front pressure mouthpiece (38) and/or a circumference of a medium receiver (10, 10a) is substantially completely adjacent to a pressure-resistant, hard boundary (19) and that preferably at least one receiver outlet (24) is located in the vicinity of a circumference of a medium receiver (10, 10a) and/or has a pressure mouthpiece (38) with roughly the same outlet cross-section as the associated smallest cross-section of a medium receiver (10).

7. Discharge apparatus according to one of the preceding claims, characterized in that at least one medium receiver (10, 10a) has at least partly hollow cross-sections, that in particular one medium receiver (10, 10a) on a side remote from a pressure mouthpiece (38) has a depression extending approximately over its entire width and that preferably a medium receiver (10, 10a) at a side remote from a pressure mouthpiece (38) is adjacent to a baffle surface (34) leading to at least one receiver outlet (24) and/or has its structure compressed with a projection pressing in a depression.

8. Discharge apparatus according to one of the preceding claims, characterized in that at least one medium receiver (10, 10a) is followed by at least one impact atomizer (30, 30a), that in particular to at least one receiver outlet (24) of a medium receiver (10, 10a) is connected a nozzle channel (23), which has at least one channel deflection (28) leading to at least one impact atomizer (30, 30a) and that preferably an impact surface (31) of an impact atomizer (30, 30a) is positioned transversely to the out-flowing flow direction and/or an impact surface (31) is located immediately upstream of a nozzle end channel (5) forming the medium outlet (4, 4a) and which has smaller passage cross-sections than the cross-sectional surface of the channel (28).

9. Discharge apparatus according to one of the preceding claims, characterized in that at least one medium outlet (4, 4a) with at least one medium receiver (10, 10a) forms at least one nozzle unit, that in particular at least one core body (8, 8a) is placed in a nozzle cap (7, 7a) traversed by a medium outlet (4, 4a) and that preferably a core body (8, 8a), which is pressure-resistant and is closer to the medium outlet (4, 4a) forms with at least one face a guiding or impact surface (34, 31) and/or a core body projecting over a rear end of the nozzle cap (7, 7a) is constructed as a medium receiver (10, 10a).

10. Discharge apparatus according to one of the preceding claims, characterized in that at least one conveying device (11, 42) has at least one source unit, which forms a structural unit with at least one discharge head (2, 2a), that in particular at least one source unit is constructed as a gas pump (43) and/or at least one source unit is constructed as a receiver pump (44) for an active medium and that preferably a receiver source (44) is connected to a medium receiver (10, 10a) and a pressure surge source (43) for the suddenly starting and substantially continuously further advancing and through-flowing fluid action on the at least partly incorporated medium on the medium receiver (10, 10a).

11. Discharge apparatus according to claim 10, characterized in that at least one pressure surge source unit (43) has a clamping device (45) for the manual pretensioning and storage of pressure surge flow energy, that in particular a pressure surge source unit (43) has a pressure gas reservoir (54) and that preferably control means (40) are provided for the substantially forced preceding of a delivery of an approximately complete discharge dose to a medium receiver (10, 10a) through a receiver source unit (44) before a pressure surge through a pressure surge source unit (43).

12. Discharge apparatus according to one of the preceding claims, characterized in that at least one conveying device (11) is operable by a screw pitch, that in particular at least one conveying device (11) is operable by turning a handle (47, 47a) and that preferably a pressure surge source unit (43, 43a) can be pretensioned by means of at least one clamping handle (47, 47a) and/or a receiver source unit (44) is operable over a discharge stroke by interposing spring damping.

13. Discharge apparatus according to one of the claims 10 to 12, characterized in that the same manual operating stroke is used on the one hand for pretensioning at least one pressure surge source unit (43) and on the other at least partly simultaneously for medium incorporation into at least one medium receiver (10, 10a), that in particular a linear stroke movement of a stroke drive narrows a pressure chamber (54) of a pressure surge source unit (43) accompanied by a pressure increase and in the force flow of the stroke drive is interposed a discharge drive for a receiver source unit (44) and that preferably at least one discharge head (2, 2a) is located in an intermediate member (51, 51a) of the discharge drive.

14. Discharge apparatus according to one of the claims 10 to 13, characterized in that at least one pressure gas source unit (43) for the suddenly starting pressure delivery can be operated from a self-secured inoperative state by at least one release handle (63, 63a), that in particular a release handle (63, 63a) is constructed as a finger key and that preferably a release handle (63, 63a) is substantially countersunk in a depression and/or is in an axis of a clamping handle (47, 47a).

15. Discharge apparatus according to one of the claims 10 to 14, characterized in that at least one pressure gas source unit (43) can be locked by means of at least one pressure outlet valve (35, 35a), that in particular at least one valve chamber outlet (38) of an outlet valve (35) is positioned immediately adjacent to a receiver inlet (21) of a medium receiver (10, 10a) and that preferably a valve chamber outlet (38) is roughly radial to the associated valve chamber (36) and/or roughly parallel to a medium receiver (10, 10a).

16. Discharge apparatus according to one of the claims 10 to 15, characterized in that at least one receiver source unit (50) is formed by a non-destructive, easily separable medium structural unit separate from at least one functional pressure gas source unit (11), that in particular a receiver source unit (50) forms a medium structural unit of self-suction pump (44) and medium reservoir (56) and that preferably an operating ram (60, 60a) of a medium structural unit can be connected by means of a plug connection to a discharge head (2 or 2a) of a pressure surge structural unit (11 or 11a) and/or a discharge head (2 or 2a) is positioned between the pressure chambers of a pressure gas source unit (11) and a medium source unit (50).

17. Discharge apparatus according to one of the claims 10 to 16, characterized in that at least one pressure gas source unit (11) and at least one receiver source unit (50) is substantially completely surrounded in a casing space of an outer casing (46), that at least one discharge head (2 or 2a) is located substantially within the casing space and that preferably a longitudinal portion of the outer casing forms a clamping handle (47) and/or a casing cover (73) for a casing opening for inserting a medium structural unit (50 or 50a).

## Revendications

1. Dispositif de distribution de produits coulants, notamment pour des distributeurs (41, 41a) à actionnement manuel, avec un corps de base (6), une sortie de produit (4, 4a) et un dispositif répartiteur (10, 10a) pour diffuser le produit, **caractérisé** en ce qu'au moins un collecteur de produit (10, 10a), pour la mise à disposition quasiment dans un état de repos de produit finement réparti, diffusé dans l'espace, est associé au dispositif répartiteur, et en ce que des moyens de refoulement (11, 11a) sont prévus pour exposer le produit mis à disposition à un nouvel écoulement en direction de la sortie de produit (4, 4a).

2. Dispositif de distribution selon la revendication 1, **caractérisé** en ce qu'il présente au moins une tête de distribution (2, 2a), sur laquelle est prévue la sortie de produit (4, 4a) débouchant vers l'extérieur, à laquelle est associée au moins un dispositif répartiteur (10, 10a) pour diffuser au moins un produit sur une étendue croissante au moyen d'un dispositif de refoulement, au moins un dispositif répartiteur présentant de préférence au moins un collecteur de produit (10, 10a) pour recevoir au moins un produit réparti et stationnaire, et au moins un collecteur de produit (10) se trouvant notamment dans le chemin d'écoulement des moyens de refoulement, comprenant au moins un dispositif de refoulement (11, 11a) à choc de pression pour décoller et entraîner au moins un produit d'au moins un collecteur de produit (10, 10a), et/ou au moins un dispositif répartiteur (10, 10a) étant conçu pour diffuser dans l'espace le produit sur un volume spatial croissant, et notamment un dispositif répartiteur présentant, à l'intérieur d'une délimitation extérieure d'au moins un collecteur de produit (10, 10a), au moins une structure de surface divisée destinée à être imprégnée par adhérence d'au moins un produit et, de préférence, au moins une structure de surface étant formée par au moins un corps collecteur (9) poreux à pores ouverts.

3. Dispositif de distribution selon la revendication 1 ou 2, **caractérisé** en ce qu'au moins un collecteur de produit (10, 10a) présentant une structure réticulée fibreuse et/ou à membrane, en ce qu'un collecteur de produit (10, 10a) est notamment comprimé à la manière d'une éponge, de façon élastiquement compressible et/ou sous précontrainte, et en ce que, de préférence, un collecteur de produit (10, 10a) est constitué au moins partiellement de matière alvéolaire de largeur de pores variable, et/ou est un corps plein essentiellement ininterrompu à l'intérieur de ses faces périphériques.

4. Dispositif de distribution selon une des revendications précédentes, **caractérisé** en ce qu'au moins un collecteur de produit (10, 10a) se trouve au moins partiellement dans la région d'une délimitation extérieure (17, 18, 19) et/ou s'applique contre cette dernière, en ce que, notamment, un collecteur de produit (10, 10a) se trouve au moins partiellement à l'intérieur d'un boîtier, et en ce que, de préférence, un collecteur de produit (10, 10a) est disposé en étant sensiblement totalement enfermé et n'est découvert que dans la région d'au moins un branchement de produit (21, 22), et/ou est limitrophe, entre ses extrémités, d'un branchement radial de produit (22).

5. Dispositif de distribution selon une des revendications précédentes, **caractérisé** en ce qu'au moins un collecteur de produit (10, 10a) présente une étendue augmentant et/ou diminuant dans une direction d'écoulement, en ce que, notamment, la section d'un collecteur de produit (10, 10a) augmente de façon approximativement constante en raccordement à au moins une entrée (21, 22) et dans la direction de passage d'écoulement d'un dispositif de refoulement (11, 11a), et en ce qu'ensuite, de préférence, la section d'un collecteur de produit (10, 10a) diminue de façon approximativement constante environ jusqu'à au moins une sortie de collecteur (24), et/ou le collecteur présente une partie (15) de section approximativement constante.

6. Dispositif de distribution selon une des revendications précédentes, **caractérisé** en ce qu'au moins une embouchure de refoulement (38) dirigée vers au moins un collecteur de produit (10) se situe dans une région de section environ minimale du collecteur de produit (10) et/ou est approximativement coaxiale à ce dernier, en ce que, notamment, un côté opposé à une embouchure de refoulement (38) située du côté frontal et/ou une face périphérique d'un collecteur de produit (10, 10a) est sensiblement en totalité limitrophe d'une délimitation dure (19) résistante à la pression, et en ce que, de préférence, au moins une sortie de collecteur (24) se trouve dans la région d'une face périphérique d'un collecteur de produit (10, 10a), et/ou une embouchure de refoulement (38) possède environ la même section de sortie que la section minimale associée d'un collecteur de produit (10).

7. Dispositif de distribution selon une des revendications précédentes, **caractérisé** en ce qu'au moins un collecteur de produit (10, 10a) présente au moins partiellement des sections creuses, en ce que, notamment, un collecteur de produit (10, 10a) présente, sur un côté éloigné d'une embouchure de refoulement (38), au moins un renfoncement s'étendant approximativement sur toute sa largeur, et en ce que, de préférence, un collecteur de produit (10, 10a) est limitrophe, sur un côté éloigné d'une embouchure de refoulement (38), d'une face déflectrice (34) menant à au moins une sortie de collecteur (24), et/ou a sa structure comprimée par une saillie imprimant un renfoncement.

8. Dispositif de distribution selon une des revendications précédentes, **caractérisé** en ce qu'au moins un pulvérisateur à rebondissement (30, 30a) est monté à la suite d'au moins un collecteur de produit (10, 10a), en ce que, notamment, un canal de buse (23) se raccorde à au moins une sortie de collecteur (24) d'un collecteur de produit (10, 10a), canal qui présente au moins une déviation de canal (28) menant à au moins un pulvérisateur à rebondissement (30, 30a), et en ce que, de préférence, une surface de rebondissement (31) d'un pulvérisateur à rebondissement (30, 30a) est transversale à la direction d'écoulement sortant, et/ou une surface de rebondissement (31) se trouve juste devant un canal terminal de buse (5) formant la sortie de produit (4, 4a), canal qui présente des sections de passage plus petites que l'aire de section du canal (28).

9. Dispositif de distribution selon une des revendications précédentes, **caractérisé** en ce qu'au moins une sortie de produit (4, 4a) forme avec au moins un collecteur de produit (10, 10a) au moins un ensemble de buse, en ce que, notamment, au moins un corps central (8, 8a) est disposé dans une calotte de buse (7, 7a) traversée par une sortie de produit (4, 4a), et en ce que, de préférence, un corps central (8, 8a) situé plus près de la sortie de produit (4, 4a) et résistant à la pression forme par au moins une face frontale une surface déflectrice ou de rebondissement (34, 31), et/ou un corps central dépassant d'une extrémité arrière de la calotte de buse (7, 7a) est conçu comme collecteur de produit (10, 10a).

10. Dispositif de distribution selon une des revendications précédentes, **caractérisé** en ce qu'au moins un dispositif de refoulement (11, 42) présente au moins une source dispensatrice qui forme une unité avec au moins une tête de distribution (2, 2a), en ce que, notamment, au moins une source dispensatrice est conçue comme pompe à gaz (43) et/ou au moins une source dispensatrice est conçue comme pompe d'alimentation (44) pour un produit actif, et en ce que, de préférence, une source d'alimentation (44) est raccordée à un collecteur de produit (10, 10a) pour emmagasiner du produit sur le collecteur de produit (10, 10a), et une source dispensatrice de choc de pression (43) est raccordée au collecteur de produit (10, 10a) pour solliciter par un fluide, d'abord par à-coups puis de façon essentiellement continue et en écoulement traversant, le produit au moins partiellement emmagasiné.

11. Dispositif de distribution selon la revendication 10, **caractérisé** en ce qu'au moins une source dispensatrice de choc de pression (43) présente un dispositif tendeur (45) pour la précontrainte manuelle et l'accumulation d'une énergie fluidique à choc de pression, en ce que, notamment, une source dispensatrice de choc de pression (43) présente un accumulateur de gaz comprimé (54), et en ce que, de préférence, des moyens de commande (40) sont prévus pour faire précéder, de façon essentiellement forcée, un choc de pression par une source dispensatrice de choc de pression (43) de la délivrance d'une dose de distribution approximativement complète à un collecteur de produit (10, 10a) par une source dispensatrice d'alimentation (44).

12. Dispositif de distribution selon une des revendications précédentes, **caractérisé** en ce qu'au moins un dispositif de refoulement (11) peut être actionné au moyen d'un pas de vis, en ce que, notamment, au moins un dispositif de refoulement (11) peut être actionné en faisant tourner une manette (47, 47a), et en ce que, de préférence, une source dispensatrice de choc de pression (43, 43a) peut être précontrainte au moyen d'au moins une manette de tension (47, 47a), et/ou une source dispensatrice d'alimentation (44) peut être actionnée avec intercalation d'un amortissement élastique sur une course de distribution

13. Dispositif de distribution selon une des revendications 10 à 12, **caractérisé** en ce qu'une seule et même course manuelle d'actionnement est prévue d'une part pour précontraindre au moins une source dispensatrice de choc de pression (43) et d'autre part, de façon au moins partiellement simultanée, pour emmagasiner du produit dans au moins un collecteur de produit (10, 10a), en ce que, notamment, un mouvement de translation linéaire d'un entraînement en translation contracte une chambre de pression (54) d'une source dispensatrice de choc de pression (43) en augmentant la pression, et un entraînement de distribution pour une source dispensatrice d'alimentation (44) est intercalé dans le chemin de force de l'entraînement en translation, et en ce que, de préférence, au moins une tête de distribution (2, 2a) est disposée dans un organe intermédiaire (51, 51a) de l'entraînement de distribution.

14. Dispositif de distribution selon une des revendications 10 à 13, **caractérisé** en ce qu'au moins une source dispensatrice de gaz comprimé (43), pour la délivrance de pression commençant par à-coups, peut être actionnée à partir d'un état de repos autobloqué par au moins une manette de libération (63, 63a), en ce que, notamment, une manette de libération (63, 63a) est conçue comme touche à doigt, et en ce que, de préférence, une manette de libération (63, 63a) se trouve essentiellement escamotée dans un renfoncement et/ou environ dans un axe d'une manette de tension (47, 47a).

15. Dispositif de distribution selon une des revendications 10 à 14, **caractérisé** en ce qu'au moins une source dispensatrice de gaz comprimé (43) peut être isolée au moyen d'au moins une soupape d'évacuation de pression (35, 35a), en ce que, notamment, au moins une sortie de chambre de soupape (38) d'une soupape d'évacuation (35) se trouve au voisinage immédiat d'une entrée de collecteur (21) d'un collecteur de produit (10, 10a), et en ce que, de préférence, une sortie de chambre de soupape (38) se trouve environ radialement à la chambre de soupape associée (36) et/ou parallèlement à un collecteur de produit (10, 10a).

16. Dispositif de distribution selon une des revendications 10 à 15, **caractérisé** en ce qu'au moins une source dispensatrice d'alimentation (50) est formée par une unité de produit qui est distincte d'au moins une source dispensatrice fonctionnelle de gaz comprimé (11) et peut en être facilement séparée sans destruction, en ce que, notamment, une source dispensatrice d'alimentation (50) forme une unité de produit constituée d'une pompe à amorçage automatique (44) et d'un réservoir de produit (56), et en ce que, de préférence, un poussoir d'actionnement (60, 60a) d'une unité de produit peut être raccordé au moyen d'un assemblage par emboîtement à une tête de distribution (2 ou 2a) d'une unité de choc de pression (11 ou 11a), et/ou une tête de distribution (2 ou 2a) se trouve entre les chambres de pression d'une source dispensatrice de gaz comprimé (11) et d'une source dispensatrice de produit (50).

17. Dispositif de distribution selon une des revendications 10 à 16, **caractérisé** en ce qu'au moins une source dispensatrice de gaz comprimé (11) et au moins une source dispensatrice d'alimentation (50) sont disposées en étant sensiblement totalement enfermées dans une chambre de boîtier d'un boîtier extérieur (46), en ce que, notamment, au moins une tête de distribution (2 ou 2a) est également disposée essentiellement à l'intérieur de la chambre de boîtier, et en ce que, de préférence, un tronçon longitudinal du boîtier extérieur forme une manette de tension (41) et/ou un couvercle de boîtier (73) pour une ouverture de boîtier destinée à l'insertion d'une unité de produit (50 ou 50a).
